(19) Europäisches Patentamt
European Patent Office
Office européen des brevets

(11) EP 3 292 861 A1

(12) EUROPEAN PATENT APPLICATION

(43) Date of publication:
14.03.2018 Bulletin 2018/11

(51) Int Cl.:
*A61K 9/127* (2006.01) *A61K 9/50* (2006.01)
*A61K 9/51* (2006.01)

(21) Application number: 16306163.3

(22) Date of filing: 13.09.2016

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
- **CENTRE NATIONAL DE LA RECHERCHE SCIENTIFIQUE -CNRS-
75016 Paris (FR)**
- **INSTITUT NATIONAL DE LA SANTE ET DE LA RECHERCHE MEDICALE (INSERM)
75013 Paris (FR)**
- **Université Paris Diderot - Paris 7
75013 Paris (FR)**

(72) Inventors:
- **PIFFOUX, Max
75013 PARIS (FR)**
- **BRUN, Amanda
91430 IGNY (FR)**
- **TARESTE, David
75013 PARIS (FR)**
- **WILHELM, Claire
78140 VELIZY-VILLACOUBLAY (FR)**
- **GAZEAU, Florence
94270 KREMLIN BICETRE (FR)**

(74) Representative: **Regimbeau
20, rue de Chazelles
75847 Paris Cedex 17 (FR)**

# (54) AN IN VITRO METHOD FOR OBTAINING A BIOHYBRID VECTOR

(57) The present invention relates to an in vitro method for obtaining a biohybrid vector comprising the step of contacting at least one extracellular vesicle (EV) with at least one liposome containing a compound of interest, wherein a fusion between the extracellular vesicle and the liposome is performed in the presence of polyethylene glycol (PEG). The present invention also relates to a biohybrid vector obtained from the method of the invention and its medical application.

EP 3 292 861 A1

## Description

## TECHNICAL FIELD OF THE INVENTION

The present invention relates to an in vitro method for obtaining a biohybrid vector comprising the step of contacting at least one extracellular vesicle (EV) with at least one liposome containing a compound of interest, wherein a fusion between the extracellular vesicle and the liposome is performed in the presence of polyethylene glycol (PEG).The present invention also relates to a biohybrid vector and its use as drug and/or for its use in medical imaging.

## BACKGROUND ART

Extracellular vesicles (EVs) are newly regarded as potential drug delivery vectors, due to their unique cellular origin and properties. These vesicles were first described in plasma in 1946 as platelet derived particles with a procoagulant activity (Chargaff et al., 1946). Since then, EVs were discovered in every biological fluid, regulating communication between cells, tissue homeostasis and accomplishing various roles in coagulation, immunity, pregnancy, infections and cancer progression.

EVs are released under physiological or pathological conditions, such as physiological shear-stress in the bloodstream (Jiang et al., 2014) that accelerates platelet derived EVs biogenesis. The first evidence of horizontal information transfer through EVs was described with the transfer of Oct4 mRNA from embryonic stem cells to bone marrow-derived mast cells (Ridder et al, 2014), and confirmed with endogenous mRNA in mast cells, endothelial cells and cancer cells. The concept of extracellular vesicular vector was really launched with the transfer of exogenous GFP mRNA in vitro (Derejibus et al. 2007) through transwell co-cultures.

The intrinsic biological activity of EVs raised therapeutic opportunities, ranging from regenerative medicine (Khan et al., 2015) to vaccination (Escudier et al., 2005). Mesenchymal stem cells-derived EVs displayed regenerative properties in various models such as myocardial infarction, brain injury, nerve remyelination or liver injury. EVs can also play an immunomodulatory role, either limiting or promoting immune responses, depending on their origin. A major potential for EVs is their use in vaccination, either in oncology or infectious disease field. For example, a vaccine against Neisseria meningitidis based on outer membrane vesicles (OMVs) released from the surface of bacteria was proven efficient in a phase I study of Shen et al. (2012). The unique properties of EVs together with their biological tolerability are now regarded as benefits for the use of EVs to deliver condensed packages of biologically active macromolecules, while protecting them from enzymes circulating in body fluids. Hacking this communication system is thus a critical issue, and an essential step for the development of novel therapies. The two major challenges for a therapeutic use are (i) the generation of EVs in sufficient quantity for clinical translation and (ii) their efficient loading with biologically active compounds such as siRNA, proteins, genes, or even viral vectors with conservation of their original content. Today, two major types of loading techniques are described, comprising biological modifications of the parent cells, or loading of EVs after their production by physical means. Regarding parent cell loading, spontaneous loading or transfection techniques have been investigated. For instance, cells were engineered to express a RVG peptide on the extracellular part of the protein lamp2b overexpressed on EVs (Alvarez-Erviti et al., 2011). This RVG peptide allowed the targeting of specific integrins. However, such strategy of transfecting parent cells with plasmids encoding the Lamp2b fused to the peptide of interest is time-consuming, challenging and may difficulty comply with a scalable Good Manifacture Practice (GMP) process. Besides, the most commonly used method for post-production EV loading is the electroporation approach. It was used to load various compounds in EVs such as siRNA (Shtam et al., 2013) DNA (Lamihhane et al., 2015), and doxorubicin (Tian et al., 2014). However, siRNA loading proved to be artefactual due to the formation of siRNA aggregates. These aggregates were co-purified with exosomes, and confounded with siRNA-loaded exosomes (Kooijmans et al., 2012).

Another method for obtaining functionalized EVs (i.e vesicles loaded with a compound of interest) consists of their destroying following by their reconstruction (Haney et al., 2015). This method is easily performable but does not allow preserving the vesicle structure thus inducing the loss of membrane and protein asymmetry with a bad rearrangement of membrane proteins. Moreover, with this method the most of loaded compound of interest is lost.

Smyth et al disclose a method for loading EVs by click chemistry. This method consists of loading the EVs membrane proteins with specific functional group in order to attach to these ones the compound of interest. However, this method does not allow loading the vesicle's lumen.

Additional method in the relating technical field is the method for loading EVs with liposomes containing the compound of interest by using freezing/thawing cycles (Sato et al.). However, this method does not allow incorporating intra-vesicular hydrophilic compounds or preserving the intra-vesicular physiological content of vesicles.

It is thus necessary to provide a method for obtaining functionalized vesicles (EVs) wherein the topology and the original proprieties (such as membrane asymmetry, endogenous loading) of vesicles are preserved but allowing modulating membrane composition and loading the vesicle's lumen. It is also necessary that this method allows loading

EVs with all kind of compounds, particularly with hydrophilic compounds, without losing the intra-vesicular physiological content of vesicles and with all kind of sample volume without losing a significant part of the compound of interest.

## SUMMARY OF THE INVENTION

In the context of the present invention, the inventors have found that in the loading process of extracellular vesicles when the fusion of EVs with liposomes containing a compound of interest is performed in the presence of polyethylene glycol (PEG), the loaded EVs preserve their topology and their original physical proprieties without destroying their membrane asymmetry or changing the protein orientation or protein structure of their membranes. The inventor have also found that the fusion of EVs with liposomes containing the compound of interest allow loading in the vesicle lumen. Moreover, it have been demonstrated that many kind of compounds, particularly, hydrophilic compounds are loaded in EVs without losing a significant quantity of the compound of interest.

The finding was unexpected since there is no motion in the prior art motivating the skilled artisan to use PEG in method for loading EVs with compounds of interest or to use it in fusion process between EVs and other structures, particularly, liposomes containing a compound of interest in order to obtain loaded EVs which may be efficiently used as biovectors (delivery systems) in various medical treatments.

It is known in the art incubating EVs with micelles in presence of PEG in order to improve the vesicles circulation in the blood (Koojimans et al., January 2016). However, it is not disclosed that the presence of PEG allows fusing EVs and liposomes containing a compound of interest in order to obtain an efficient delivery system without the inconvenient of the delivery systems of the prior art.

Moreover, Lee et al (1997) disclose the fusion of vesicles with another vesicles or the fusion of liposomes with another liposomes in presence of PEG but these fusions are fusions between symmetrical structures whereas in the present invention the fusion of the extracellular vesicles (EVs) with the liposomes is a fusion between a asymmetric structures from which fusion results a hybrid structure.

In first aspect, the present invention thus relates to an *in vitro* method for obtaining a biohybrid vector comprising the step of contacting at least one extracellular vesicle (EV) with at least one liposome containing a compound of interest, wherein a fusion between the extracellular vesicle and the liposome is performed in the presence of polyethylene glycol (PEG).

The inventors have demonstrated in the experimental part that the biohybrid vector obtained by the method of the invention consisting of an extracellular vesicle fused with a liposome containing a compound of interest, has the same physiological proprieties as the EV before fusion and the topology of its membrane as well as the structure are preserved. Moreover, it contains significant quantity of the compound of interest. This demonstration asserts that the biohybrid vector obtained by the method of the invention possess specific advantageous characteristics.

In second aspect, the present invention thus relates to a biohybrid vector obtained by the method of the invention consisting of an extracellular vesicle fused with a liposome containing a compound of interest.

The inventors have further demonstrated that it is possible to assess the efficiency of fusion reaction via the dilution pairing lipids FRET (present in the liposome before the fusion reaction) and thus it is possible to control and adapt the conditions of the fusion reaction in order to obtain a biohybrid vector having the required characteristics. In such a way, it is possible to provide a biohybrid vector having the required characteristics in order to be used efficiently as a drug delivery system in a medical treatment.

In third aspect, the present invention thus relate to a biohybrid vector obtained by the method of the invention for use as drug in various medical treatments.

Particularly, the biohybrid vector of the invention may be used in regenerative medicine, in genetic and cell therapy, in cancer therapy, in immunotherapy and in vaccination.

The inventors have also demonstrated that the biohybrid vector according to the invention may be used in medical imaging.

## DESCRIPTION OF THE FIGURES

**Figure 1: PEG-mediated fusion between magnetic EVs and liposomes monitored by the FRET-based lipid mixing assay.** Non-fluorescent magnetic EVs and fluorescent liposomes consisting of 67% DOPC, 30% PE, 1.5% NBD-PS and 1.5% Rhodamine-PE were incubated in the absence or the presence of 5% (w/v) PEG 8000. The increase of NBD fluorescence reflects the dilution of NBD-PS and Rhodamine-PE in the fused EV/liposome membrane. **A**-**B**- Fusion assay at 50°C of microvesicles (MV) or exosomes (Exo) with liposomes using a ratio lipo/$EV_{(MV\ or\ Exo)}$ = 1/9 (A) or 1/1 (B). **C-D-** Fusion assay at 50°C with and without dithionite treatment and lipo/EV = 1/1 (C) and at 130 min (D). **E-** Fusion assay at 40°C for 24 hours of total EV population with liposomes using a

ratio lipo/EV = 1/1. Results in A and B show one representative experiment. Results in D and E are expressed as mean ± SEM with N = 3 independent EV preparations.

**Figure 2: PEG-mediated fusion between magnetic EVs and liposomes using different concentration of PEG.** Non-fluorescent magnetic EVs and fluorescent liposomes consisting of 67% DOPC, 30% PE, 1.5% NBD-PS and 1.5% Rhodamine-PE were incubated at 50 °C during 2h with a 1:1 ratio in the absence or the presence of 2,5, 5, 7,5 and 10% (w/v) PEG 8000. The increase of NBD fluorescence reflects the dilution of NBD-PS and Rhodamine-PE in the fused EV/liposome membrane. Fusion efficiency is correlated to PEG 8000 concentration (from 0 to 10 %). Results are expressed as mean ± SEM with N=6 independent EV preparations for PEG 5% and no PEG, and N=3 for the others.

**Figure 3: PEG-mediated fusion between magnetic EVs and liposomes at different fusion temperatures.** Non-fluorescent magnetic EVs and fluorescent liposomes consisting of 67% DOPC, 30% PE, 1.5% NBD-PS and 1.5% Rhodamine-PE were incubated between 4 and 60 °C during 2h with a 1:1 ratio in the absence or the presence of 5 % (w/v) PEG 8000. The increase of NBD fluorescence reflects the dilution of NBD-PS and Rhodamine-PE in the fused EV/liposome membrane. Fusion efficiency is correlated to temperature increase. Results are expressed as mean ± SEM with N=6 independent EV preparations for peg 50°C and 50°C no PEG, and N=3 for the others

**Figure 4: PEG-mediated fusion between magnetic EVs and liposomes with different molecular mass of PEG.** Non-fluorescent magnetic EVs and fluorescent liposomes consisting of 67% DOPC, 30% PE, 1.5% NBD-PS and 1.5% Rhodamine-PE were incubated at 50 °C during 2h with a 1:1 ratio in the absence or the presence of 5 % (w/v) PEG from 3000 to 8000 MW. The increase of NBD fluorescence reflects the dilution of NBD-PS and Rhodamine-PE in the fused EV/liposome membrane. Fusion efficiency is correlated to PEG molecular weight. Results are expressed as mean ± SEM with N=6 independent EV preparations for peg 8000 and no PEG, and N=3 for the others

**Figure 5: PEG-mediated fusion between magnetic EVs and liposomes wherein the liposomes contain polar lipids compared to non polar (neutral lipids).** The fusion is performed between EVs and liposomes (with PEG or without PEG, respectively black or gray) containing positive lipids (EPC 30 % DOPE 30% POPC 40%) (square) and between EVs and neutral lipids (POPC 70%/ DOPE 30%) indicated in the figure as "normal lipids" (triangles). during 2h in ratio 1:1, 100μl, 50 °C, 5% PEG 8000, N=3.

**Figure 6: PEG-mediated fusion between magnetic EVs and liposomes enriched in DOPE-biotinylated lipids, to allow coupling to desired streptavidin labelled components post fusion.** Non-fluorescent magnetic EVs and fluorescent liposomes consisting of 66% DOPC, 30% PE, 1.5% NBD-PS and 1.5% Rhodamine-PE and 1 % $DOP_{12}$-biotin were incubated at 50 °C during 2h with a 1:1 ratio in the absence or the presence of 5 % (w/v) PEG 8000 MW. The increase of NBD fluorescence reflects the dilution of NBD-PS and Rhodamine-PE in the fused EV/liposome membrane. Biotinylated lipids does not limit fusion. Results are expressed as mean ± SEM with N=6 independent EV preparations for PEG 8000 and no PEG, and N=3 for the others.

**Figure 7: Separation of liposomes and EVs using density gradient ultracentrifugation to quantify the transfer of fluorescent lipids, hydrophilic load or siRNA from liposomes to EVs. A-** Fluorescence profile of a suspension of PKH26 labelled EVs and of rhodamine liposomes in the density gradient after ultracentrifugation. Three zones have been defined: 0-0.75 mL encompassing mostly liposomes, 1-3 mL corresponding to EVs with higher density than liposomes, and 3.25-5 mL corresponding to the leaking zone. **B**- Rhodamine fluorescence profile of non-fluorescent EVs fused with liposomes composed of 67% PC, 30% PE, 1.5% NBD-PS and 1.5% Rhodamine-PE in the absence or presence of PEG for 120 min at 50°C in using a 9/1 lipo/EV ratio. Note the transfer of rhodamine-PE fluorescence from the liposome zone to the EV zone after fusion in the presence of PEG **(inset). C-** Percent of rhodamine lipid fluorescence in the liposome zone, EV zone and leaking zone for different liposome/EV ratios (9/1 and 1/1), demonstrating that about 23± 9 % of the total lipid fluorescence was lost in the liposome zone and completely transferred to the EVs zone after PEG-induced fusion. **D-** Percent of hydrophilic rhodamine fluorescence in the liposome zone, EV zone and leaking zone after fusion of rhodamine-loaded liposomes (composed of 67% POPC and 30% PE liposomes, and loaded with 2 μM sulforhodamine B) with non-fluorescent EVs. After PEG-induced fusion, the EV zone displays 30% of total rhodamine fluorescence. In comparison to the fusion without PEG, the fluorescence loss of the liposome zone (21,7 ± 4,5 % of total fluorescence) in the presence of PEG is redistributed in the EV zone (13,1 ± 2,1 % of the total fluorescence) and in the leaking zone (9,5 ± 2,5% of the total fluorescence). **E-**Percent of siRNA fluorescence in the liposome zone, EV zone and leaking zone after fusion of siRNA-loaded liposome (consisting of 67% PC and 30% PE, and loaded with 2 μM rhodamine siRNA) with non-fluorescent EVs. After PEG-induced fusion, the EV zone displays 30% of total siRNA-rhodamine fluorescence. In

comparison to the fusion without PEG, the fluorescence loss of the liposome zone (21.6 ± 2 % of total fluorescence) in the presence of PEG is redistributed in the EV zone (16 ± 2 % of total fluorescence) and in the leaking zone (5,5 ± 2,4% of the total fluorescence).

**Figure 8: Micromagnetophoresis and Nanoparticle Tracking Analysis (NTA) confirm PEG-induced EV/Liposome fusion without any loss of EV's magnetic internal cargo.** Iron oxide nanoparticles-loaded non-fluorescent EVs and fluorescent liposomes were co-incubated with or without PEG during 120 min at 50°C with a liposome/EV ratio of 1/1 and further separated using density gradient ultracentrifugation. The 1.75-2 mL fraction (most populated EV zone) was analysed with micromagnetophoresis and NTA. **A-**Representative pictures of the magnetic tip following accumulation of magnetic EVs after fusion with rhodamine fluorescent liposomes, once captured on the magnet, EVs were washed to remove any unspecific liposome capture. (bright field picture on the left and rhodamine fluorescence picture on the right, scale bar represents 10 μm). **B-**Fluorescence intensity on the magnetic tip after PEG-mediated fusion of magnetic EVs with liposomes composed of 67% PC, 30% PE, 1.5% NBD-PS and 1.5% Rhodamine-PE displayed an 11-fold increase compared to the signal measured without PEG. **C-** The absolute concentration of fluorescent objects in the 1.75-2 mL EV zone counted by NTA after fusion of EVs with membrane labeled liposomes was 5-fold higher in the presence of PEG. **D-** The proportion of fluorescent objects among all objects counted by NTA in the 1.75-2 mL EV zone after fusion of EVs with membrane labeled liposomes was 9-fold higher in the presence of PEG. **E-** Fluorescence intensity on the magnetic tip after EV fusion with liposomes encapsulating Sulforhodamine B displayed in a 6-fold increase compared to the signal measured in the absence of PEG. Results in B and C, D and E are expressed as mean ± SEM, n = 3. * represents $p < 0.05$, ** $p < 0.01$, and *** $p < 0.001$ compared with groups at the same time point using student T test.

**Figure 9: Imaging flow cytometry analysis of fusion mix after fusion.** Magnetic non-fluorescent EVs and fluorescent liposomes were co-incubated with or without PEG during 120 min at 50°C with a liposome/EV ratio of 1/1. Liposomes composed of 67% PC, 30% PE, 1.5% NBD-PS and 1.5% Rhodamine-PE were detected and imaged using their rhodamine fluorescence. EVs were detected and imaged using annexin V labeling. BioHybrid EVs are considered as events positive both for rhodamine and anxV (double positive events). Fluorescence intensity, size and picture of each event were recorded using Image Stream X flow cytometer. **A-** Fluorescence intensity of double positive events (biohybrid EVs) after fusion with or without PEG. After fusion in presence of PEG, the rhodamine fluorescence of double positive events is nearly 9 fold higher (liposome) than without PEG. **B**- Percentage of double positive events among annexin V positive events. After fusion in presence of PEG, about 80 % of annexin V positive events (EVs) are rhodamine positive (i.e. contain content from a liposome) versus 35 % without PEG. **C-**Size distribution of EVs alone (annexin V positive events, rhodamine negative) and ofEVs fused with liposomes (double positive events) for 2h in presence of PEG or without PEGin comparison to calibration beads. Fusion with liposomes in presence of PEG induces a shift toward larger sizes of EVs. Results in A, B and C are expressed as mean ± SEM, n = 3. * represents $p < 0.05$, ** $p < 0.01$, and *** $p < 0.001$ compared with groups at the same time point using student T test.

**Figure 10. Cryo TEM pictures of the fusion mix after fusion between EVs and liposomes.** Non-fluorescent EVs and fluorescent liposomes consisting of 66% DOPC, 30% PE, 1.5% NBD-PS and 1.5% Rhodamine-PE and 1 % DOPE-biotin produced by extrusion with a 50 nm filter were incubated at 40 °C during 2h with a 1:1 ratio in the absence or the presence of 10 % (w/v) PEG 8000 MW. Mix was then diluted 10 times in HEPES buffer supplemented with 2mM calcium. 10 nm annexin V gold nanoparticles and 14 nm streptavidin gold nanoparticles were added to the mix and incubated for 15 min prior preparation for cryo TEM. Pictures A, B, C and D are representative pictures of double labelled biohybrids. Fig E represents representative pictures of liposomes incubated with 14 nm streptavidin gold nanoparticles, not incubated with PEG, Fig F represents representative pictures of EVs incubated with 10 nm annexin V gold nanoparticles, not incubated with PEG,

**Figure 11** represents the size distribution in cryo TEM of Extracellular vesicles alone (n=145), liposomes alone (n=43), the mixture of both in presence of PEG for 2h (n=117), and only double positive vesicles (n=32).

## DETAILED DESCRIPTION OF THE INVENTION

### *An in vitro method for obtaining biohybrid vector*

In the context of the present invention the inventors have found surprisingly that contacting at least one extracellular vesicle (EV) with at least one liposome containing a compound of interest in presence of polyethylene glycol (PEG) allows obtaining an EV (or also called herein biohybrid vector) containing a part of liposome membrane lipids

and proteins and almost the full quantity of the compound of interest. The inventors have demonstrated that due to the presence of PEG during the fusing possess the obtained biohybrid vector conserve its content without significant changes, particularly the asymmetry and the topology of its membrane remain.

This finding is very important since the use of PEG which is easy and not expensive allows obtaining a functional biohybrid vector which may be efficiently used for medical purpose.

In the first aspect, the present invention thus relates to an *in vitro* method for obtaining a biohybrid vector comprising the step of contacting at least one extracellular vesicle (EV) with at least one liposome containing a compound of interest, wherein a fusion between the extracellular vesicle and the liposome is performed in the presence of polyethylene glycol (PEG).

In the present description the term **"extracellular vesicle"** refers to membrane-vesicles released in an evolutionally conserved manner by cells ranging from organisms such as prokaryotes to higher eukaryotes and plants. The significance of EVs lies in their capacity to transfer information to other cells thereby influencing the recipient cell function. EV-mediated signals can be transmitted by all the different biomolecule categories: protein, lipids, nucleic acids and sugars. The unique package of this information provides both protection and the option of simultaneous delivery of multiple different messengers even to sites remote to the vesicular origin.

According to one embodiment of the method of the invention, the extracellular vesicles are selected from the group comprising a microvesicle, an exosome and an apoptotic body.

In the context of the invention, the terms **"microvesicle"** or **"microparticles"** or **"exosome"** relate to one of three main classes of extracellular vesicles (EVs) wherein the vesicles are produced by outward budding and fission of the plasma membrane.

In the context of the present invention, the term **"exosome"** relates to the extracellular vesicles that are formed within the endosomal network and released upon fusion of multi-vesicular bodies with the plasma membrane.

In the context of the present invention, the term **"apoptotic body"** relates to the extracellular vesicles released as blebs of cells undergoing apoptosis.

According to the preferred embodiment of the method of the invention, the extracellular vesicles are selected from the class of microvesicles or exosomes.

In the method of the present invention, at least one extracellular vesicle (at least one microvesicle and/or at least one exosome and/or at least one apoptic body) is contacted with at least one liposome containing at least one compound of interest in the presence of PEG.

In the context of the invention, the term **"liposome"** relates to spherical vesicle having at least one lipid bilayer composed of phospholipids, especially phosphatidylcholine. When the liposomes are constituted by only one phospholipid bilayer, they are called *unilamellar Liposome vesicles*; otherwise they are called *multilamellar vesicles*. In the relating art, the term liposome also refers to artificial vesicles.

According to one embodiment of the method of the invention, the liposome contains a lipid medium comprising polar (also called herein positive lipids) or non polar lipids of any kind. In one embodiment, the liposomes are constituted from phospholipids. Preferably the phospholipids are selected from the group comprising phosphatidyl ethanolamine POPC, DOPC, $DOP_{12}$-biotin, DOPS-NBD, $DOP_{13(orB)}$-Rho, $DOP_{13}$-NBO. More preferably, the phospholipides used for manufacturing liposomes in the context of the invention is POPC.

It was demonstrated in the present invention that these liposomes have very good ability to fusion with EVs in presence of PEG. The skilled artisan knows which lipid should be selected and which concentration is appropriated in order to manufacture a liposome suitable for medical use after its fusion with EVs.

More preferably, in the present invention the liposomes are manufactured with non polar lipids, also called "normal" or "physiological" "lipids" which are able to easily fuse with membrane lipid of EVs in presence of PEG. A mean advantage of these lipids is that they have no toxic effect, compared to polar lipids that tends to be toxic.

The liposomes used in the method of the invention may be obtained by any conventional method used in the related art. The skilled artisan knows how to modulate the conditions of a liposome manufacturing method in order to obtain liposomes having specific lipid content.

In the method of the inventions at least one EV is fused with at least one liposome containing a compound of interest. The compound of interest may be any compound used for medical purposes.

According to one embodiment, the compound of interest is at least one selected from the group comprising a nanoparticle, a supramolecular assembly, a hydrophobic molecule, a hydrophilic molecule and an amphiphilic molecule.

In the context of the invention, the term **"nanoparticles"** refers to any particles having a size from 1 nm to 1000 nm. Examples of nanoparticles which may be a compound of interest according to the invention are solid lipid nanoparticles, polymeric particles, metal nanoparticles, nanotubes, iron oxide nanoparticles, gold nanoparticles, multilayer nanoparticles, dendrimers, quantum dots.

In the context of the invention, the term **"supramolecular assembly"** or "**supermolecule**" relates to a well-defined complex of molecules held together by noncovalent bonds. The supramolecular assembly can be simply composed of two molecules (e.g., a DNA double helix or an inclusion compound) or larger complexes of molecules that form

sphere-, rod-, or sheet-like species. The dimensions of supramolecular assemblies can range from nanometers to micrometers.

Particularly, in the present invention a supramolecular assembly may be an assembly between proteins or an assembly between a nanoparticle and a specific layer (X layer) surrounding the nanoparticles.

In the context of the invention the term **"hydrophobic molecule"** refers to a molecule which tends to be non-polar and, thus, prefer other neutral molecules than water and non-polar solvents. Hydrophobic molecules in water often cluster together, forming micelles. Water on hydrophobic surfaces will exhibit a high contact angle. Examples of hydrophobic molecules include the alkanes, oils, fat, and greasy substances in general. Often the hydrophobic molecule is also a lipophilic molecule.

In the context of the invention, the term **"hydrophilic molecule"** relates to molecule or portion of a molecule that is attracted to, and tends to be dissolved by water. Their interactions with water and other polar substances are more thermodynamically favorable than their interactions with oil or other hydrophobic solvents. They are typically charge-polarized and capable of hydrogen bonding. This makes these molecules soluble not only in water but also in other polar solvents.

In the context of the invention the term **"amphiphilic molecule"** refers to a molecule possessing both hydrophilic (*water-loving,* polar) and lipophilic (*fat-loving*) properties. Such a molecule is also called *amphiphilic* or *amphipathic.* Common amphiphilic are lipoproteins.

According to one embodiment of the invention, the compound of interest is selected from the group comprising a hydrophobic molecule selected from the group comprising waxes, fats, oils, alkanes, membrane proteins, a hydrophilic molecule selected from the group comprising colloids, polar covalent compounds like alcohols such as $C_2H_5OH$ (ethanol) and ketones like $(CH_3)_2C=O$ (acetone), sugars, ionic compounds like KCl, nucleic acids, amino acids, and phosphate esters, calcium chloride, $CaCl_2$, non steroid hormones, glucose, insulin, mineral acids and bases, and a amphiphilic molecule selected from the group comprising phospholipids, glycolipids, cholesterol, pepducins, fatty acids, bile acids, saponins, local anaesthetics.

Preferably, the compounds of interest used in the method of the invention are hydrophilic molecules selected from the group comprising siRNA, miRNA, DNA, proteins.

In this regard, the method of the invention is particularly advantageous since the method for obtaining functionalized vesicles known in the prior art do not allow easily loading of extracellular vesicles with hydrophilic molecules.

According to the method of the invention, the fusion between a least one EV and at least one liposome containing a molecule of interest is performed in presence of polyethylene glycol (PEG).

In the context of the invention the term **"polyethylene glycol"** or **"PEG"** refers to a polyether compound (also known as polyethylene oxide (PEO) or polyoxyethylene (POE)). The structure of PEG is commonly expressed as $H-(O-CH_2-CH_2)_n-OH$ or by the following structural formula:

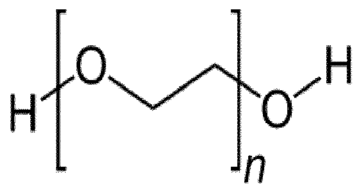

*Polyethylen glycol (PEG)*

According to one embodiment of the method of the present invention, PEG may have a molecular weight selected from 3000 to 15000 g/mol. More particularly, PEG used in the method of the invention has a molecular weight from 3000 to 10000 g/mol, even more particularly PEG has a molecular weight from 3000 to 8000 g/mol. In the most preferred embodiment of the method of the invention PEG has a molecular weight of 8000 g/mol.

According to another embodiment, the method of the invention may be performed in presence of different concentrations of PEG. Preferably, the concentration of PEG is comprised in the range of 2 to 15 (weight/volume). More preferably, PEG concentration is comprised between 5% w/v and 10% w/v of total weight of all reacting components and even more preferably the PEG concentration is 10% w/v of all reacting components.

In one embodiment of the method of the invention, the fusion between a least one EV and at least one liposome containing a molecule of interest is performed at temperature comprised in range between 30°C and 70°C, preferably, between 37°C and 45°C. More preferably this fusion is performed at 40°C.

In another embodiment of the method of the invention, the fusion between a least one EV and at least one liposome containing a molecule of interest is performed in large range ratio vesicle/liposome determined in accordance with the nature of the compound of interest. For example, when the compound of interest is difficult to obtain or expensive, the number of EVs is significantly higher than the number of liposomes containing the compound of interest. Particularly, the ratio vesicle/liposome may range between 1/1 to 1/1000 or 1/1 to 1000/1. Preferably, in the method of the present

invention the ratio vesicle/liposome is between 9/1 to 1/1, more preferably this ratio is 1/1.

The method of the invention may be performed by combining various parameters such as the concentration and the molecular weight of PEG, the fusion temperature, the physiological content of liposomes and the ratio vesicles/liposomes. Specific examples of combinations are given in the experimental part of the present application. Preferably, when the concentration of PEG is 5% or 10% (weight/volume), the molecular weight of PEG is 8000 g/mol, the fusion temperature is 50°C and the ratio vesicles/liposomes ranges between 9/1 to 1 /1.

In one embodiment of the method of the invention liposomes may be enriched with other molecules before their fusion with extracellular vesicles. Particularly, liposomes may be enriched with biotinylated lipids, allowing grafting of any streptavidin linked compound after.

Alternately, the liposomes may be enriched with pH sensitive lipids, allowing localized delivery, fusogenic lipids to enhance fusion, a targeting ligand to allow the targeting of a key tissue/cell in the body, a imaging agent, to allow the imaging follow up of the biohybrid

In another embodiment, the method of the invention may comprise an additional step of purifying the biohybrid vector. Preferably, following to EV/liposome fusion, it is possible to isolate the fused EV (biochybrid vectors), based on their density, by density gradient ultracentrifugation.

Alternately, the purification methods may be a simple ultracentrifugation, affinity chromatography, size chromatography, or magnetic sorting.

Moreover, the method of the invention may comprise a step of removing non fused liposomes and EVs.

Alternately, in case of high efficiency fusion, purification of biohybrid vectors from non-fused liposomes and extracellular vesicles is not needed, in this case, removal of PEG with dialysis is a method of interest.

***A biohybrid vector obtained by the method of the invention***

The inventors have demonstrated that the biohybrid vector obtained by the method of the invention consisting of an extracellular vesicle fused with a liposome containing a compound of interest has the same physiological properties as the EV before fusion and the asymmetry of its membrane as well its structure is preserved. Moreover, it contains significant quantity of the compound of interest. This demonstration asserts that the biohybrid vector obtained by the method of the invention possess specific advantageous characteristics.

In the second aspect, the present invention thus relates to a biohybrid vector consisting of an extracellular vesicle fused with a liposome containing at least one compound of interest.

In the context of the invention, the term **"biohybrid vector"** relates to an extracellular vesicles fused with a liposome wherein the biohybrid vector contains its own membrane structure (lipids and proteins) and another physiological content being in the extracellular vesicle before fusion but the biohybrid vector also contain membrane proteins and lipid from liposome and the molecule of interest. The membrane of biohybrid vector may be transformed at the same times as the inner part of the vector allowing thus modulating the delivery capacity of this biohybrid vector

***Applications of the biohybrid vector obtained by the method of the invention***

The inventors have further demonstrated that it is possible to assess the efficiency of fusion reaction via the dilution pairing lipids FRET (present in the liposome before the fusion reaction and thus it is possible to control and adapt the conditions of the fusion reaction in order to obtain a biohybrid vector having the required characteristics. In such a way, it is possible to provide a biohybrid vector which may be used efficiently as a drug delivery system in a medical treatment.

In third aspect, the present invention thus relate to a biohybrid vector obtained by the method of the invention for use as drug in various medical treatments.

The biohybrid vector of the invention may be used for developing vesicular therapy in order to replace cell therapy and thus overcoming its inconvenient such as auto immune reaction risk of teratoma, sample storage, etc.

Drug delivery ability of the biohybrid vector of the invention may be generally used in oncology. The immunostimulatory or regulatory properties of this biohybrid vector can be applied to immunotherapies or vaccination.

In the field of the vaccination, the biohybrid vector of the invention may be particularly used for the production of AAV viral vectors encapsulated in an extracellular vesicle, allowing both loading the target and increasing protection against antibodies against AAV present in 50% of patients.

In the context of the invention, the term **"AAV" or "Adeno-associated virus"** relates to a member of the parvovirus family, which is composed of small viruses with a genome of a single-stranded DNA. AAVs insert genomic material at a specific site on human chromosome 19 with nearly 100% certainty, and are used to construct vectors that introduce genes into cultured cells or human beings for gene therapy.

According to one embodiment, the biohybrid vector of the invention is used as drug and/or drug delivery system.

Particularly, the biohybrid vector is used in immunotherapy, genetic and cell therapy, oncology and vaccination.

More particularly, the biohybrid vector of the invention is used as drug for treating disease selected from auto-immune diseases, degenerative diseases, metabolic disease, infectious diseases and cancer.

In the context of the invention, the term **"auto-immune disease"** relates to a pathological state arising from an abnormal immune response of the body to substances and tissues that are normally present in the body. Examples of such disease are Type I diabetes, Psoriasis, Alopecia areata, Antiphospholipid antibody syndrome (aPL), Autoimmune hepatitis, Celiac disease, Graves' disease, Guillain-Barre syndrome, Hashimoto's disease, Hemolytic anemia, Idiopathic thrombocytopenic purpura, Inflammatory bowel disease (IBD), Inflammatory myopathies, Myasthenia gravis, Primary biliary cirrhosis, Rheumatoid arthritis, Scleroderma, Sjögren's syndrome, Systemic lupus erythematosus, Vitiligo.

In the context of the invention, the term **"degenerative diseases"** relates to medical problems that worsen over time. These degenerative diseases may affect the central nervous system (brain and spinal cord), bones, blood vessels or heart. Examples of degenerative diseases are diseases selected from the group comprising or consisting of diseases affecting the central nervous system (Alzheimer's disease and Parkinson disease, Huntington diseases), bones (Duchene and Becker muscular dystrophies), blood vessels or heart.

In the context of the invention the term **"metabolic diseases"** relates to any of the diseases or disorders that disrupt normal metabolism. Metabolic diseases affect the ability of the cell to perform critical biochemical reactions that involve the processing or transport of proteins, carbohydrates, or lipids. Examples of metabolic disorders include diabetes, in particular type 1 or type 2 diabetes, obesity, syndrome X, impaired glucose tolerance, impaired fasting glucose, gestational diabetes, maturity-onset diabetes of the young (MODY), latent autoimmune diabetes adult (LAD A), associated diabetic dyslipidemia, hyperglycemia, hyperinsulinemia, dyslipidemia, hypertriglyceridemia, and insulin resistance.

In the context of the invention, the term **"infectious diseases"** relates to any disease or disorder caused by an infection with a microorganism. The infection may be caused by all type of microorganism, particularly by bacterium, viruses fungi, yeasts etc.

As used herein, the terms **"cancer"** and **"cancerous"** refer to or describe the physiological condition in mammals that is typically characterized by unregulated cell growth. The terms "cancer" and "cancerous" as used herein are meant to encompass all stages of the disease. Thus, a "cancer" as used herein may include both benign and malignant tumors.

In one embodiment of the invention, the cancer being treated is osteolysis, bone sarcomas (osteosarcoma, Ewing's sarcoma, Giant cell tumours of bone), bone metastases, glioblastoma and brain cancers, lung cancer, acoustic neuroma, acute leukemia, acute lymphocytic leukemia, acute myelocytic leukemia (monocytic, myeloblastic, adenocarcinoma, angiosarcoma, astrocytoma, myelomonocytic and promyelocytic), acute T-cell leukemia, basal cell carcinoma, bile duct carcinoma, bladder cancer, breast cancer, bronchogenic carcinoma, cervical cancer, chondrosarcoma, chordoma, choriocarcinoma, chronic leukemia, chronic lymphocytic leukemia, chronic myelocytic (granulocytic) leukemia, chronic myleogeneous leukemia, colon cancer, colorectal cancer, craniopharyngioma, cystadenocarcinoma, diffuse large B-cell lymphoma, dysproliferative changes (dysplasias and metaplasias), embryonal carcinoma, endometrial cancer, endotheliosarcoma, ependymoma, epithelial carcinoma, erythroleukemia, esophageal cancer, estrogen-receptor positive breast cancer, essential thrombocythemia, fibrosarcoma, follicular lymphoma, germ cell testicular cancer, glioma, heavy chain disease, hemangioblastoma, hepatoma, hepatocellular cancer, hormone insensitive prostate cancer, leiomyosarcoma, liposarcoma, lung cancer, lymphagioendotheliosarcoma, lymphangiosarcoma, lymphoblastic leukemia, lymphoma (Hodgkin's and non-Hodgkin's), malignancies and hyperproliferative disorders of the bladder, breast, colon, lung, ovaries, pancreas, prostate, skin and uterus, lymphoid malignancies of T-cell or B-cell origin, leukemia, lymphoma, medullary carcinoma, medulloblastoma, melanoma, meningioma, mesothelioma, multiple myeloma, myelogenous leukemia, myeloma, myxosarcoma, neuroblastoma, non-small cell lung cancer, oligodendroglioma, oral cancer, osteogenic sarcoma, ovarian cancer, pancreatic cancer, papillary adenocarcinomas, papillary carcinoma, pinealoma, polycythemia vera, prostate cancer, rectal cancer, renal cell carcinoma, retinoblastoma, rhabdomyosarcoma, sarcoma, sebaceous gland carcinoma, seminoma, skin cancer, small cell lung carcinoma, solid tumors (carcinomas and sarcomas), small cell lung cancer, stomach cancer, squamous cell carcinoma, synovioma, sweat gland carcinoma, thyroid cancer, Waldenstrom's macroglobulinemia, testicular tumors, uterine cancer and Wilms' tumor.

In one particular embodiment, the biohybrid vector of the invention may be used as vaccine.

As used herein, the term **"vaccine"** relates to a biological preparation that provides active acquired immunity to a particular disease. A vaccine typically contains an agent that resembles a disease-causing micro-organism and is often made from weakened or killed forms of the microbe, its toxins or one of its surface proteins. The agent stimulates the body's immune system to recognize the agent as a threat, destroy it, and keep a record of it, so that the immune system can more easily recognize and destroy any of these micro-organisms that it later encounters. Vaccines can be prophylactic (example: to prevent or ameliorate the effects of a future infection by any natural or "wild" pathogen), or therapeutic (e.g., vaccines against cancer).

The biohybrid vector of the invention may also be used in pharmaceutical composition. The present invention thus relates to a pharmaceutical composition comprising a biohybrid vector obtained from the method of the invention and pharmaceutically acceptable vehicle.

In the context of the present invention, the term **"pharmaceutical acceptable vehicle"** refers to a compound, or a combination of compounds, entering a pharmaceutical composition that does not cause secondary reactions and that, for example, facilitates administration of the active compounds, increases its lifespan and/or effectiveness in the organism, increases its solubility in solution or improves its storage. Such pharmaceutical vehicles are well-known and will be adapted by a person skilled in the art according to the nature and the administration route of the biohybrid vector.

According to another embodiment of the invention, the biohybrid vector may be used in medical imaging. Particularly, its use comprises encapsulating a contrast medium in the biohybrid vector or attaching a contrast molecule to biohybrid vector through a ligand. Examples of such methods are use of hydrophobic contrast agent, coupling to the surface using chemical reaction, or coupling with a receptor ligand interaction.

Any other method of medical imaging well known in the relating art may be used with the biohybrid vector of the invention. Example of this method is surface absorption.

Further characteristics and embodiments will be apparent from the Examples which follow and from the figures.

## Examples

### *Example 1 : Materials and methods*

Materials

1,2-dioleoyl-sn-glycero-3-phosphocholine (DOPC), 1-palmitoyl-2-oleoyl-*sn*-glycero-3-phosphocholine (PC), 1,2-dioleoyl-sn-glycero-3-phosphoethanolamine (PE), 1,2-dioleoyl-*sn*-glycero-3-phospho-L-serine (PS), 1,2-distearoyl-*sn*-glycero-3-phosphoethanolamine-N-(7-nitro-2-1,3-benzoxadiazol-4-yl) (PE-NBD), 1,2-dioleoyl-*sn*-glycero-3-phospho-L-serine-N-(7-nitro-2-1,3-benzoxadiazol-4-yl) (PS-NBD), 1,2-dioleoyl-*sn*-glycero-3-phosphoethanolamine-N-(lissamine rhodamine B sulfonyl) (PE-Rho) were purchased from Avanti Polar lipids as chloroform solutions. DDM (ULTROL grade) and Dithionite (grade for analysis) were purchased from Merck. PEG8000 (BioUltra grade), Sulforhodamine B (laser grade) and PKH26 were purchased from Sigma-Aldrich. SiRNA Cy5 labeled was purchased from Eurogentech (Angers). Citrate-coated magnetic iron oxide ($\gamma$Fe2O3) 9 nm nanoparticles were kindly provided by Christine Menager (Phenix, UPMC) and produced by alkaline co-precipitation of $FeCl_2$ and $FeCl_3$ salts, according to Massart's procedure (Massart et al, 1981). Their molarity was measured by atomic emission spectroscopy. Nycodenz was purchased from Progen biotechnik (Heidleberg, Germany).

Preparation of liposomes

Liposomes were prepared using the extrusion method. 1 $\mu$mol of the appropriate lipid mixture (in chloroform) was dried in a glass tube for 10 min under a gentle stream of argon, and evaporation was continued for 2 h under vacuum to remove all trace of organic solvent. The dried lipid film was hydrated by adding 1 mL of PBS (including 1-2 $\mu$M of cargoes for cargo-loaded liposomes) to obtain a final lipid concentration of 1 mM, and the solution was vortexed vigorously for 1 hour at room temperature. Multi-lamellar vesicles were frozen in liquid nitrogen (~30 sec) and then thawed in a 40°C water bath (~5 min); the sequence was repeated 7 times. Small unilamellar vesicles (SUVs) were obtained by extrusion through a 0.1 or 0.05 $\mu$m polycarbonate membrane using the Avanti Mini-Extruder; at least 19 passages were performed. Cargo-loaded liposomes were purified from non-encapsulated cargoes by flotation in a Nycodenz step gradient (as described above in the method part).

Cell culture and nanoparticle loaded EV isolation

Human umbilical vein endothelial cells HUVEC were cultured at 37 °C and 5% $CO_2$ in Dulbecco's Modified Eagle Medium (DMEM) supplemented with 10% fetal bovine serum (FBS) and 100 U/mL penicillin and 100 U/mL streptomycin. To trigger EV release, cells were cultured in serum-free DMEM for 3 days. EVs were isolated from the conditioned culture medium with a differential (ultra)centrifugation method as previously described by Théry et al., 2006). First cell debris were eliminated by 2,000 $\times$g centrifugation for 10 min. From the supernatant, microvesicles (MVs) were isolated after the 10,000 $\times$g ultracentrifugation step for 20 min and exosomes (Exos) were further isolated after the final 100,000 xg step (1 hour). Alternatively the total population of extracellular vesicles (EVs, containing both microvesicles and exosomes) were isolated with a simple 100,000 $\times$g step for 1 hour without 10,000 $\times$g step. Vesicles were then resuspended in phosphate-buffered saline (PBS).

To produce magnetic EVs loaded with iron oxide nanoparticles, HUVEC cells were first incubated with citrate-coated iron oxide nanoparticles (5 mM iron in serum-free RPMI medium supplemented with 5 mM sodium citrate) during 2 hours at 37 °C, as previously described (Rautou et al, 2012). Incubation was followed by 2 washing steps in serum-free RPMI medium and 2 hours chase in serum-supplemented DMEM medium in order to allow nanoparticle internalization. The cells were further cultured in serum free culture medium to trigger EV release as described above.

Fusion of liposomes with EVs using PEG and monitoring using FRET-based lipid-mixing assay

Liposomes and EVs were mixed in Nunc F96 MicroWell White plates. The total volume of the reaction mixture was 120 $\mu$L. EVs and liposomes were mixed in each well, with a liposome/EV ratio of 1/1 (2 x $10^{10}$ objects), 1/9 (2 x $10^{10}$ objects) or 9/1 (2 x $10^{11}$ objects) in PBS. Objects were counted with Nanoparticle Tracking Analysis, as described below. PEG 8000 was added or not at a final concentration of 5 % (w/v). PBS and PEG 8000 were heated before mixing at the appropriate temperature. Plates were maintained at 50°C in the SpectraMax M5 plate reader (Molecular Devices). Alternatively, for the fusion assay at 40°C, the volume of the reaction mixture was 240 $\mu$L and the temperature was maintained in the ThermoMixer (Eppendorf). Lipid mixing during EV-liposome fusion was monitored by Fluorescence Resonance Energy Transfer (FRET) as described previously (Scott et al., 2003). Briefly, donor fluorescent liposomes were prepared containing equal molar ratio of the fluorescent lipids NBD-PS and Rho-PE. Upon excitation of NBD, energy is transferred to rhodamine in a process known as FRET, which is strongly dependent on the distance between the two fluorophores. Therefore, dilution of the NBD-PS and Rho-PE lipids within the membrane of the acceptor non fluorescent EVs (when donor liposomes and acceptor vesicles fuse) results in an increase in NBD fluorescence. Lipid mixing was monitored by following the NBD fluorescence intensity (excitation at 460 nm, emission at 535 nm, cut-off at 530 nm). After 2 hr, the fusion reaction was stopped by the addition of 10 $\mu$L of DDM at 2.5% (w/v) to solubilize all liposomes and thus measure the NBD fluorescence intensity at infinite dilution, Max(NBD). Fusion curves were normalized using the following equation :

$$\text{NBD Fluorescence Increase (\%)} = [\text{NBD} - \text{Min(NBD)}] / [\text{Max(NBD)} - \text{Min(NBD)}] \quad \text{(equation 1)}$$

Where Min(NBD) is the lowest fluorescence value from all time points.

The NBD fluorescence increase was related to the number of rounds of fusion per donor liposome using the equation established by (Parlati et al., 1999) for the fusion of donor and acceptor liposomes.

$$\text{Round of fusion} = (0.49666 * e^{(0.036031*X)}) - (0.50597 * e(^{-0.053946}*X)) \text{ (equation 2)},$$

where X is the percentage of NBD fluorescence increase for any time point of the kinetic measurement.

As indicated above, all experiments are performed using the follow up of FRET fluorescence increase during time, which is correlated to dilution of the FRET pair fluorophores from the liposome in the Extracellar vesicles membrane. The higher fluorescence increase = the higher fusion occurs, for example:

- 5 % fluorescence increase = 0,21 fusion per liposome
- 10 % fluorescence increase = 0,42 fusion per liposome
- 20 % fluorescence increase = 0,85 fusion per liposome
- 30 % fluorescence increase = 1,36 fusion per liposome
- 40 % fluorescence increase = 2,04 fusion per liposome

Fusion of liposomes with EVs using different concentrations of PEG and monitoring using FRET-based lipid-mixing assay

To assess the fusion between EVs and liposomes at different concentrations of PEG liposomes and EVs were mixed in closed Eppendorf 155 mL tubes and incubated at the desired temperature. The total volume of the reaction mixture was 20 $\mu$L. EVs and liposomes were mixed in each well, with a liposome/EV ratio of ratio 1/1 (POPC 67%/DOPE 30%/ 1.5% DOPS-NBD, 1.5% $DOP_{13(orB)}$-Rho) in PBS. Objects were counted with Nanoparticle Tracking Analysis, as described below. PEG 8000 was added or not at a final concentration of 2.5%, 5%, 7.5% and 10% (w/v). PBS and PEG 8000 were heated before mixing at the appropriate temperature. Eppendorf 1.5 mL tubes were heated at 50°C in a water bath or Eppendorf Thermomixer . Lipid mixing during EV-liposome fusion was monitored by Fluorescence Resonance Energy Transfer (FRET) as described previously.

Fusion of liposomes with EVs using PEG at different temperatures of fusion and monitoring using FRET-based lipid-mixing assay

To assess the fusion between EVs and liposomes at different temperatures of fusion, liposomes and EVs were mixed in closed Eppendorf 1.5 mL tubes and incubated at the desired temperature. The total volume of the reaction mixture was 20 μL. EVs and liposomes were mixed in each well, with a liposome/EV ratio of ratio 1/1 (POPC 67%/DOPE 30%/ 1.5% DOPS-NBD, 1.5% $DOP_{13(orB)}$-Rho) in PBS. Objects were counted with Nanoparticle Tracking Analysis, as described below. PEG 8000 was added or not at a final concentration of 5% (w/v). PBS and PEG 8000 were heated before mixing at the appropriate temperature. Eppendorf 1.5 mL tubes were heated either at 37°C, 40°C, 45°C, 50°C or 60°C in a water bath or Eppendorf Thermomixer. Lipid mixing during EV-liposome fusion was monitored by Fluorescence Resonance Energy Transfer (FRET) as described previously.

Fusion of liposomes with EVs using PEG with different molecular weight and monitoring using FRET-based lipid-mixing assay

To assess the fusion between EVs and liposomes at different concentrations of PEG liposomes and EVs were mixed in closed Eppendorf 1.5 mL tubes and incubated at the desired temperature. The total volume of the reaction mixture was 20 μL. EVs and liposomes were mixed in each well, with a liposome/EV ratio of ratio 1/1 (POPC 67%/DOPE 30%/ 1.5% DOPS-NBD, 1.5% $DOP_{13(orB)}$-Rho) in PBS. Objects were counted with Nanoparticle Tracking Analysis, as described below. PEG having molecular weight of either 3000, 6000 or 8000 was added or not at a final concentration of 5% (w/v). PBS and PEG 8000 were heated before mixing at the appropriate temperature. Eppendorf 1.5 mL tubes were heated either at 50°C in a water bath or Eppendorf Thermomixer. Lipid mixing during EV-liposome fusion was monitored by Fluorescence Resonance Energy Transfer (FRET) as described previously.

Fusion of liposomes containing positive/polar lipids with EVs using PEG and monitoring using FRET-based lipid-mixing assay compared to neutral lipids

To assess the fusion between EVs and liposomes containing positive or polar lipids, liposomas and EVs were mixed in Nunc F96 MicroWell White plates. The total volume of the reaction mixture was 100 μL. EVs and liposomes were mixed in each well, with a liposome/EV ratio of ratio 1/1 (EPC 30% DOPE 30% POPC 37%/ 1.5% DOPS-NBD, 1.5% $DOP_{13(orB)}$-Rho) or (POPC 670%/DOPE 30%/ 1,5% DOPS-NBD, 1.5% $DOP_{13(orB)}$-Rho) in PBS. Objects were counted with Nanoparticle Tracking Analysis, as described below. PEG 8000 was added or not at a final concentration of 5% (w/v). PBS and PEG 8000 were heated before mixing at the appropriate temperature. Plates were heated either at 50°C in the SpectraMax M5 plate reader (Molecular Devices). Lipid mixing during EV-liposome fusion was monitored by Fluorescence Resonance Energy Transfer (FRET) as described previously.

Fusion of liposomes enriched in DOPE-biotinylated lipids with EVs using PEG using FRET-based lipid-mixing assay

To assess the fusion between EVs and liposomes enriched in DOPE-biotinylated lipids, liposomes and EVs were mixed in closed Eppendorf 1.5 mL tubes and incubated at the desired temperature. The total volume of the reaction mixture was 20 μL. EVs and liposomes were mixed in each well, with a liposome/EV ratio of ratio 1/1 (POPC 66%/DOPE 30% / DOPE biotinylated lipid 1% / 1.5% DOPS-NBD, 1.5% $DOP_{13(orB)}$-Rho) in PBS. Objects were counted with Nanoparticle Tracking Analysis, as described below. PEG 8000 was added or not at a final concentration of 5% (w/v). PBS and PEG 8000 were heated before mixing at the appropriate temperature. Eppendorf 1.5 mL tubes were heated either at 50°C in a water bath or Eppendorf Thermomixer. Lipid mixing during EV-liposome fusion was monitored by Fluorescence Resonance Energy Transfer (FRET) as described previously.

Dithionite assay to probe for hemifusion vs. full fusion

To assess the possibility of hemifusion between liposomes and vesicles, fluorescent donor liposomes were preincubated with 0.15 mM sodium dithionite for 10 min at 37°C and then transferred to 4°C before mixing with acceptor vesicles. The efficiency of NBD reduction to nonfluorescent N-(7-amino-2,1,3-benzoxadiazole- 4-yl) was estimated to be between 50% and 60% of the total NBD-PS content. In addition, dithionite was shown to completely loose its activity after 10 min at 37°C, ensuring that NBD-PS from the inner leaflets are not quenched following DDM (n-Dodecyl beta D maltoside) addition. By measuring the total lipid-mixing and inner leaflet-mixing fluorescence signals independently, the percentage of hemifused vesicles (Ph) could be calculated using the following equation:

$$Ph = (Pt - Pi)/(Pt - f^*Pi)^*100 \text{ (equation 3)},$$

where Pt is the percentage of NBD fluorescence increase for total lipid mixing, Pi is the percentage of surface of lipids coming from inner leaflet lipid mixing and f is the proportion of lipids contained in the inner layer of the liposome (with 100 nm liposomes : f = 0.45).

PKH26 labeling of EVs

EVs were incubated with 1 μM fluorescent lipophilic tracer PKH26 with provided buffer at room temperature prior to density gradient ultracentrifugation. Fluorescence was quantified using Molecular Devices SpecM5 plate reader (Excitation at 560 nm, Emission at 595 nm, filter at 590 nm).

Purification and separation of EVs and fused EVs from liposomes with density gradient ultracentrifugation

A discontinuous gradient of Nycodenz was made by use of 0, 15 and 80% Nycodenz solutions in PBS. The sample to purify was mixed with 80% Nycodenz solution in a 1/1 ratio, to obtain a 40% mixed solution. 3 mL of this 40% mixture, 1.5 mL of 15% Nycodenz in PBS, and 0.5 mL of pure PBS solutions were put on top of each other in a 6 mL open top Ultra-clear centrifuge tube (Beckman Coulter) and centrifuged in a SW 32.1 Ti rotor (Beckman Coulter) at 246,000 g (45,000 RPM) for 4 hours at 4°C. Gradient fractions of 0.25 mL were collected from the top of the gradient, and the fluorescence was quantified for each of these 20 fractions using the SpectraMax M5 plate reader (Excitation at 560 nm, Emission at 595 nm, cut-off at 590 nm for rhodamine; Excitation at 650 nm, Emission at 670 nm, cut-off at 665 nm for Cyanine 5 labelled siRNA).

Micromagnetophoresis

For micromagnetophoresis of iron oxide loaded EVs, 30 μL of EVs samples were inserted in a glass slide/coverslip chamber featuring an integrated nickel microrod. When the microrod was magnetized by a permanent magnet, the local magnetic field generated at its extremity attracted magnetic objects toward the micromagnet, covering the tip. The nickel microrod was submitted to a 150 mT uniform magnetic field from a rectangular magnet positioned aside. Micromagnetophoresis chambers were observed by means of an Olympus JX81 / BX61 Device/Yokogawa CSU Device spinning disk microscope (Andor Technology plc, Belfast, Northern Ireland) equipped with a 60X Plan-ApoN oil objective lens (60X/1.42 oil, Olympus). The excitation wavelength for Rhodamine was 405 nm and a filter at 685 nm was used for collecting fluorescence emission. Image J software was used to follow fluorescence on the tip of the micromagnetophoresis by selecting the tip zone on the bright field picture, and quantifying the fluorescence on the corresponding zone on the fluorescent picture. Each sample was analysed with 3-4 tips, and the mean value was calculated for each sample.

Nanoparticle tracking analysis

EV size distribution and concentration were determined with nanoparticle tracking analysis (NTA) using a Nanosight LM10-HS (NanoSight, UK) with a 532 nm laser. Before measurements, EVs were diluted to an appropriate dilution with sterile PBS (confirmed to be particle-free). Of each sample, 10 movies of 12 s were recorded using camera level 16. Fluorescent EVs among all EVs were quantified using a filter to eliminate the light from non-fluorescent objects (laser light was filtered, only emitted light from the fluorophore was detected). Data was analyzed with NTA Analytical Software suite. For counting EVs and liposomes, one movie of 60 s was recorded using camera level 16. It has to be taken into account that the number of EVs seen by NTA is highly depending on the purity of the sample. In our case, EVs were produced in serum-free culture medium, therefore limiting the protein aggregates contamination. With this EV generation method, the purity ratio in particle/μg of proteins is $2^*10^{10}$p/ug, corresponding to a pure sample.

Image Stream X flow cytometer

Image Stream X® imaging flow cytometer (Amnis) was used for high throughput imaging of liposomes, EVs and biohybrids and for determining their size distribution, and fluorescence intensity. Briefly, rhodamine was used to detect liposomes, Annexin V was used to detect labeled EVs, and double positive events were considered to be biohybrids. Annexin V labeling was performed using manufacturer instruction (ROCHE, annexin V fluo, FITC). Samples were diluted 1000 times before use. Calibration beads were used as a way to determine the size of vesicles. Regions of interest were determined using appropriate negative controls.

Cryo TEM pictures of the fusion mix after fusion between EVs and liposomes

Non-fluorescent EVs and fluorescent liposomes consisting of 66% DOPC, 30% PE, 1.5% NBD-PS and 1.5% Rhodamine-PE and 1 % $DOP_{12}$-biotin produced by sonication were incubated at 40 °C during 2h with a 1:1 ratio in the absence or the presence of 10 % (w/v) PEG 8000 MW. Mix was then diluted 10 times in HEPES buffer supplemented with 2mM calcium. 10 nm annexin V gold nanoparticles (kindly provided by Alain Brisson) and 14 nm streptavidin gold nanoparticles (Sigma) were added to the mix (1/10 dilution) and incubated for 15 min prior preparation for cryo TEM.

Confocal pictures of internalization of fluorescent biohybrids on cell cultures

Non-fluorescent magnetic EVs and fluorescent liposomes consisting of 67% DOPC, 30% PE, 1.5% NBD-PS and 1.5% Rhodamine-PE were incubated at 50 °C during 2h with a 1:1 ratio (10^11 objects) in the presence of 5 % (w/v) PEG 8000 MW and Biohybrids were purified using ultracentrifugation with Nycodenz gradient to remove any unfused liposome. CT26 and Huvec cells were then incubated with the resulting biohybrids for 18 hours, washed with PBS, fixed and stained with DAPI. Cells were then imaged using a confocal microscope in bright field, DAPI fluorescence (cell nucleus) and Rhodamine fluorescence (Biohybrids).

Statistical data analysis

Statistics Data are presented as standard deviation from the mean ($n \geq 3$). Student t-test carried out to determine a significant difference between two groups using Prism 3.0 version of GraphPad software (USA). A minimum of 95% confidence level was considered significant (*** indicates $P < 0.0001$; ** indicates $P < 0.01$; * indicates $P < 0.05$ and NS means not statistically significant).

***Exemple 2 : Resul ts***

Fusion of liposomes with exosomes and microvesicles in the presence of PEG.

The inventors explored the parameters related to EV/Liposome fusion to find the optimal conditions for EV engineering. Protein-free membrane fusion can be triggered *in vitro* using chemical agents with the capacity to mediate close-contact and dehydration of lipid bilayer structures.

100 nm-diameter liposomes were produced with a "physiological" concentration of PE (PC 70 %, PE 30%) in order to minimize changes in the EV lipid composition after fusion. The fusion process was monitored using a previously described FRET-based lipid mixing assay. Briefly, donor liposomes containing FRET pairs of fluorescent lipids (1.5% NBD-PE or NBD-PS and 1.5% Rhodamine-PE) dilute their lipids in the acceptor vesicle membrane during fusion, which results in the increase of the mean distance between NBD and rhodamine and thus leads to a lower FRET efficiency. The increase of NBD fluorescence thus allows following the fusion process over time.

Magnetic microvesicles (MVs) or exosomes (Exos) subpopulations of EVs were incubated with fluorescent liposomes composed of 67% DOPC, 30% PE, 1.5% NBD-PE and 1.5% Rhodamine-PE (mol percents) in the absence or presence of 5% (weight/volume) PEG 8000 during 90 min at 50°C, using a ratio of 9 vesicles per liposome (Fig. 1A) or 1 vesicle per liposome (Fig. 1B). Liposomes fused at a similar speed with both Exos and MVs, as shown by the same linear increase of NBD fluorescence during the incubation time of 90 min. The fusion rate was 8 fold higher in the presence of PEG. With the ratio of 9 EVs per liposome, NBD fluorescence increased to about 20% of its maximum value with Exos and 25% with MVs, corresponding respectively to 0.49 and 0.58 rounds of fusion per liposome after 90 min, using the calibration equation described in the method section. This means that approximately half of the liposomes have fused with one EV. With the ratio of 1 EV per liposome, NBD fluorescence increased to 8% with Exos and 10% with MVs, corresponding respectively to 0.2 and 0.25 rounds of fusion per liposome. Taking into account the liposome/EV ratio, the probability for one Exo or one MV to fuse with one liposome was therefore larger with the 1/1 ratio (about 25% vs. 1/9=11% in the case of the 1 /9 ratio) and was similar with Exos and MVs.

Further fusion experiments were then performed in the presence of PEG with equal numbers of liposomes and EVs (ratio 1/1) and with the total EV fraction from the 2000-100 000g pellet containing both Exos and MVs (Fig. 1C and D). Indeed, exosomes (Exos) and microvesicles (MVs) are produced by the same cells, in the same time, and their physical properties are similar. Classical isolation procedures are indeed selecting EVs depending on their size and density, which is overlapping a lot. Once proved that both exosomes and microvesicles were able to fuse in the same way, we decided to merge both (EVs) for the rest of the experiences. As fusion is a temperature dependent mechanism, we also investigated the fusion between liposomes and EVs at lower temperature. EVs were incubated at 40°C for 24 hours in equal molar ratio with liposomes composed of 67% PC, 30% PE, 1.5% NBD-PS and 1.5% Rhodamine PE. This temperature was described by Koojimans SA et al. (2013) as a non-degradative temperature for exosomal proteins.

Under these conditions, the fusion process is slowed down by a factor of 10 compared to the experiments performed at 50°C. However the fluorescence increase is still linear, even after 24 hours, reaching 5.8% of the maximum NBD fluorescence and corresponding to 0.15 rounds of fusion (Fig. 1 E).

EV/liposome fusion is complete, without hemifusion.

We used the dithionite assay to assess if the fusion between liposomes and EVs is complete or only involves the fusion of outer monolayers (hemifusion). Incubation of fluorescent liposomes with dithionite is quenching the NBD fluorescence signal in the outer monolayer of the liposome. Any fluorescence dequenching measured by the FRET-based lipid mixing assay with dithionite treated liposomes is therefore the result of inner monolayer fusion, and the signature of full fusion between liposomes. We used fluorescent liposomes consisting of 67% PC, 30%, PE, 1.5% NBD-PS and 1.5% Rhodamine PE because the rate of NBD-PS flip-flop is extremely low and totally negligible under our experimental conditions. The fusion curves obtained between EVs and either dithionite-treated or non-treated liposomes (Fig. 1D and E) are very similar (+/-1% error). The percentage of hemifused vesicles (Ph) calculated using equation (3) converges to zero, demonstrating the complete fusion of liposomes.

Fusion of liposomes with exosomes and microvesicles in the presence of PEG at different concentration of PEG

Magnetic microvesicles (MVs) or exosomes (Exos) subpopulations of EVs were incubated with fluorescent liposomes composed of 67% POPC and 30% DOPE and 1.5% Rhodamine-PE (mol percents) in the absence or presence of either 2.5%, 5%, 7.5% or 10% (weight/volume) PEG 8000 during 120 min at 50°C, using a ratio of 1 vesicles per liposome. As shown on Fig 2, the fusion rate was 8 fold higher in the presence of PEG at 10% than without PEG and the fusion rate was 3.5 fold higher with PEG at 10% than with PEG at 2.5%.

Fusion of liposomes with exosomes and microvesicles in the presence of PEG at different temperatures

Magnetic microvesicles (MVs) or exosomes (Exos) subpopulations of EVs were incubated with fluorescent liposomes composed of 67% POPC and 30% DOPE and 1.5% Rhodamine-PE (mol percents) in the absence or presence of 5%, (weight/volume) PEG 8000 during 120 min at either 37°C, 40°C, 45°C, 50°C or 60°C, using a ratio of 1 vesicles per liposome. As shown on Fig 3, the fusion is efficient from 37°C to 60°C. The high variability in the 60°C is due to the nonspecific fusion due to draying in the 60°C condition.

Fusion of liposomes with exosomes and microvesicles in the presence of PEG having different molecular weight

Magnetic microvesicles (MVs) or exosomes (Exos) subpopulations of EVs were incubated with fluorescent liposomes composed of 70% POPC and 30% DOPE and 1.5% Rhodamine-PE (mol percents) in the absence or presence of 5%, (weight/volume) PEG having molecular weight of either 3000, 6000 or 8000 during 120 min at 50°C, using a ratio of 1 vesicles per liposome. As shown on Fig 4, the fusion in presence of PEG is more efficient than the fusion without PEG. Moreover, Fig. 4 shows that PEG8000 allow obtaining better fusion than PEG 3000 and PEG6000. It is also shown that there is no significant difference on the fusion between PEG3000 and PEG6000.

Fusion of liposomes containing positive or polar lipids with EVs using PEG and monitoring using FRET-based lipid-mixing assay

Magnetic microvesicles (MVs) or exosomes (Exos) subpopulations of EVs were incubated with fluorescent liposomes composed of either (EPC 30% DOPE 30% POPC 40%) or (POPC 70%/DOPE 30%) and 1.5% Rhodamine-PE (mol percents) in the absence or presence of 5%, (weight/volume) PEG8000 during 120 min at 50°C, using a ratio of 1 vesicles per liposome. Fig. 5 shows that positive lipids in allow better fusing between EVs and liposomes containing them compared to the liposomes containing "normal' physiological lipids. However, Fig. 5 clearly shows that the liposomes containing "normal" lipids allow fusing with EVs much more better in presence of PEG than in absence of PEG.

Fusion of liposomes enriched in DOPE-biotinylated lipids with EVs in the presence of PEG by FRET-based lipid-mixing assay

Magnetic microvesicles (MVs) or exosomes (Exos) subpopulations of EVs were incubated with fluorescent liposomes enriched in DOPE-biotinylated lipids and 1.5% Rhodamine-PE (mol percents) in the absence or presence of 5%, (weight/volume) PEG8000 during 120 min at 50° C, using a ratio of 1 vesicles per liposome. Fig. 6 shows that fusion is efficient with liposome enriched in DOPE-biotinylated lipids allowing coupling streptavidin labelled component on

biohybrid EVs post fusion.

EVs and fused liposomes-EVs can be separated from liposomes using density gradient centrifugation

Lipid mixing assay allowed investigating the process of fusion from the membrane point of view. The inventors next investigated the efficiency of cargo transfer from liposomes to EVs. Indeed, some fusion mechanisms are inducing the loss of the inner content such as freeze-thawing or extrusion. Assays can also be used to monitor content mixing. Indeed, as an example, internal hydrophilic dilution during fusion proved by the EDTA/ Calcein + $Co^{2+}$ couple requires one liposome loaded with EDTA and one liposome loaded with both Calcein and $Co^{2+}$. Once fused, EDTA chelates $Co^{2+}$, which is not able to stop Calcein fluorescence anymore. However, in our case, loading of the acceptor EV with a compound is needed, which is not possible. The inventors decided to separate liposomes and EVs using a density gradient, and quantify the fluorescence in each zone to prove the transfer of molecules from liposomes to EVs.

The inventors compared the fluorescence distribution of a solution of fluorescent liposomes with that of PKH26 labelled EVs in a discontinuous density gradient of Nycodenz constituted by 3 layers as illustrated in Fig. 7A. The fluorescence of liposomes with a density of 1 is retrieved in the first fractions from the zone 0-1.5 mL (with a majority, 94 %, in the zone 0-0.75 mL). By contrast, PKH26-labeled EVs distribute in the 0.5-3 mL zone, with a peak between the fractions 1.5 and 2 mL. This is consistent with the previously reported higher density of EVs (1.15) compared to liposomes (1.03). According to these distributions, we have defined three zones, the first one (0-0.75 mL) to separate the major part of liposomes, the second one (1-3 mL) to retrieve EVs, and the third one (3.25-5 mL) defined as the leaking zone.

Loading of EV membrane with liposome membrane compounds of interest

The proof of principle of EV membrane engineering is done with fluorescent lipids incorporated into liposome membrane but is applicable to membrane proteins or drugs.

Using the EV-liposome separation method described previously, the inventors verified the efficiency of fluorescent lipid transfer from liposomes (67% PC, 30% PE, 1.5% NBD-PS and 1.5% Rhodamine-PE) to non-fluorescent EVs by fusion in the presence or absence of PEG, and assessed both the reliability of their density gradient centrifugation measurement and their ability to transfer lipids of interest to EVs. The inventors fused magnetic EVs and fluorescent liposomes at 50°C during 2 hours using a liposome/EV ratio of 1/1 or 9/1. They then separated these samples with the density gradient, and measured the rhodamine fluorescence along the gradient (Fig. 7B for the condition liposome/EV=9/1 and liposome/EV=1/1) in the three zones described above (Fig. 7C). Compared to the control condition without PEG, about 20% (23,6 and 21,7 % respectively) of the fluorescence was redistributed from the liposome zone (0-0.75 mL) to the EVs zone (1-3 mL) in both cases (Ratio 9 /1 and 1 /1). No fluorescence (<1%) was detected in the leaking zone (3.25-5 mL). It may be thus concluded that, in the presence of PEG, 20% of the fluorescence coming from liposomal lipids has been transferred to EVs. This result is consistent with the 1/1 FRET fusion assay reported above to demonstrate PEG-induced lipid transfer to the EV fraction. In the case of the 9/1 condition, this corresponds to the fusion of about 2 liposomes per EV (23,6 % of 9 liposomes), and in the case of 1/1 condition, it corresponds to the fusion of about 0,21 (21% of 1 liposome) liposome per EV, confirming the previous results using lipid mixing assay.

Loading of EVs with internal hydrophilic cargo by fusion with loaded liposomes

Similarly to the lipid transfer, the inventors tested the transfer efficiency of hydrophilic cargo encapsulated into liposomes to EVs upon PEG-induced fusion. Rhodamine-loaded liposomes or Cy5 siRNA-loaded liposomes (concentration under the quenching zone) were fused with non-fluorescent EVs at 50°C during 2 hours using a 1/1 ratio (Fig. 7D&E). Compared to the control without PEG, 22% of the rhodamine fluorescence disappeared from the liposome zone (0-0.75 mL) ; 13% of this fluorescence was redistributed to the EVs zone (1-3 mL) and 9% to the leaking zone (3.25-5 mL). These results are consistent with previous experiments investigating the lipid transfer result (about 20% transfer). We can calculate that about 60 % (13/[13+9]) of the transferred hydrophilic molecules were loaded in the EVs whereas 40 % leaked during the process. Leakage is an inherent feature of our liposome preparation as shown by the results obtained in the absence of PEG and it is increased during fusion in the presence of PEG (Fig. 7D). Similarly, 16% of total siRNA fluorescence was transferred to EVs in the presence of PEG with only 6% loss in the leaking zone (72 % efficiency). Despite these losses due to leakage PEG-mediated fusion is efficient for transferring soluble compounds from liposomes to EVs. It is possible that the leaking process depends on the size or nature of the content, with bigger compounds such as siRNA (7 x 2 x 2 nm, lower diffusion coefficient than rhodamine) showing less leakage.

Using these results, it is concluded that with the 1/1 ratio, for which around 0,21 liposomes (100 nm) fused with each vesicle (140 nm), an average of 4-5 % volume of the biohybrid vector is composed of liposome content. If we hypothesize that the leaking percentage is the same in the 9/1 ratio, 25-30 % volume of the biohybrid vector is composed of liposome content. Moreover, the size of the liposome can be tuned to favor the loading.

Micromagnetophoresis and NTA proove that EVs conserve their previous internal cargo, and confirms fusion.

The inventors assessed the conservation of EV loading and its fusion with liposomes with micromagnetophoresis and NTA. Micromagnetophoresis is a technique allowing the observation of magnetic nanoparticles loaded vesicles, by attracting them on a magnet. Once attracted, the aggregation of nano-objects on the magnet can easily be seen and quantified. NTA is allowing the visualization of nano-objects down to 40 nm, by quantifying their Brownian motion.

Liposome bearing fluorescent lipids in their membrane or encapsulating soluble rhodamine were fused at a 1/1 ratio with EVs loaded with iron oxide nanoparticles using PEG for 2 hours at 50°C. Samples were then separated on the density gradient. The 1.75-2 mL portion of the gradient, corresponding to the middle of the EV zone, was selected for micromagnetophoresis assay. Micromagnetophoresis allows selective attraction of nanoparticle-loaded EVs on the micromagnet tip, while non-magnetic nanocontainers (liposomes or EVs) remain in suspension (Fig. 8A). Fluorescence in the area of the magnet tip was 11-fold higher when fusion was performed with PEG compared to the condition without PEG using liposomes labeled with fluorescent lipids (Fig. 8B) and 6-fold higher using rhodamine-loaded liposomes (Fig 8E). Such increase in fluorescence confirms that magnetic nanoparticle-loaded EVs conserve their intrinsic cargo (magnetic nanoparticles as tracers but also probably RNA, proteins or metabolites), while acquiring lipids and internal cargo of liposomes upon fusion in the presence of PEG.

The 1.75-2 mL fraction was also analyzed by NTA with the standard scattering mode (monitoring the Brownian motion of all scattering particles) or with the fluorescence mode (using a filter to detect only fluorescent particles). The concentration of fluorescent objects after fusion with PEG ($2.8\times10^9$) is 5-fold higher than without PEG ($5.7\times10^8$, Fig. 8D), and the percentage of fluorescent objects among all objects is 9-fold higher (64% versus 7%) with PEG than without PEG (Fig. 8E). The diameter of the total population of objects was not significantly changed by PEG-induced fusion (Table 1). However, fluorescent objects corresponding to fused EVs were significantly larger than the overall population (226 versus 128 nm), which is consistent with an increase of EVs size during fusion with liposomes.

**Table 1 :** NTA characterization of particles in the 1.75-2 mL fraction of the density gradient after fusion of EVs with membrane labeled liposomes

| | **With PEG Fluorescent mode** | **With PEG Standard mode** | **Without PEG Fluorescent mode** | **Without PEG Standard mode** |
|---|---|---|---|---|
| **Concentration (number/mL)** | 2.8E+09 (+/- 1.1E+09) | 4.3E+09 (+/- 1.4E+09) | 5.7E+08 (+/- 1.0E+08) | 1.1E+10 (+/- 4.8E+09) |
| **Mean diameter (nm)** | 226 (+/- 57) | 128 (+/- 1) | 177 (+/- 68) | 133 (+/- 24) |

Image Stream X flow cytometer confirms fusion

The inventors used Imaging Flow cytometry as a way to confirm EVs/liposomes fusion. The goal is to image EVs, liposomes and biohybrid EVs and characterize the fluorescence emission and size of each detected object (event). Iron oxide nanoparticles-loaded non-fluorescent EVs and fluorescent liposomes were co-incubated with or without PEG during 120 min at 50°C with a liposome/EV ratio of 1/1. Liposomes composed of 67% PC, 30% PE, 1.5% NBD-PS and 1.5% Rhodamine-PE were detected using their rhodamine fluorescence. EVs were detected using an annexin V labeling. Hybrid EVs are considered to be double positive events (Figure 9) (Fluorescence intensity, size and picture of each event was recorded using Image Stream X flow cytometer (10000 events). After fusion in presence of PEG, around 80 % of the Annexin V positive objects (EVs) are rhodamine positive i.e. contain content from a liposome), versus around 35 % without PEG. Moreover the rhodamine fluorescence of double positive events (biohybrid EVs) is nearly 9 fold higher after fusion in presence of PEG compared to without PEG. Finally, fusion in presence of PEG induces a shift toward larger sizes of biohybrid EVs in comparison to the precursor EVs and to biohybrid EVs obtained in absence of PEG.

Cryo TEM pictures of the fusion mix after fusion between EVs and liposomes confirms the fusion event

To ensure that Liposomes and EVs were not destroyed by PEG during the process of fusion, and do not lose their integrity or surface properties and orientation, EVs and biotin liposomes were fused for 2h at 40 °C in 10% PEG, stained for specific markers and observed in Cryo TEM. Briefly, EVs were labeled with 10 nm annexin V gold nanoparticles (kindly provided by Alain Brisson) and biotin liposomes were stained with 14 nm streptavidin gold nanoparticles (Sigma). It was observed double positive vesicles, that were considered as biohybrid vectors (Figure 10, A, B, C, D). We compared the distribution in diameter of EVs alone, liposome alone, the average size of all vesicles (liposomes and vesicles not differentiated) and double positive events (Figure 11). Liposomes (61 +/- 36 nm) and EVs (123 +/- 79 nm) diameter was

clearly different, but after mixing, this difference disappeared to obtain an average diameter slightly bigger than EVs alone (143 +/- 104 nm). The difference was even more visible when looking specifically at double positive vesicles (199 +/- 139 nm). This increase in size is consistent with the fusion process, and biohybrids keep a vesicle morphology, and are not destroyed by the process.

Furthermore, the inventors incubated fluorescent biohybrid vectors with different cell lines (HUVEC and CT26) to confirm their ability to be internalized. Hence, it appears that biohybrid conserve the EV property to be internalyzed by cells, notably tumor cells (CT 26).

Confocal pictures (data not shown) of internalization of fluorescent biohybrid cell cultures confirms internalization and conservation of EV properties.

***Conclusion***

In light of different experimental results obtaining in this invention, it appears that:

- PEG can strongly stimulate the fusion between EVs and liposomes comprising a compound of interest;
- Fusion is accelerated at high temperature (about 50°C);
- PEG-mediated fusion between EVs and liposomes is complete without detectable hemifusion;
- EVs preserve their initial content;
- 99% of liposome lipids fused to biohybrids are found in the biohybrids without leaking, and more than 60% of hydrophilic the molecule of interest;
- EVs may be loaded with different type of compounds, particularly with hydrophilic compounds (siRNA or free rhodamine), and with lipophilic compounds.
- Biohybrid vector (EVs fused with liposome containing a compound of interest) may be efficiently used as drug delivery system.

**BIBLIOGRAPHIC REFERENCES**

- Al Faraj A, Gazeau F, Wilhelm C, Devue C, Guérin CL, Péchoux C, Paradis V, Clement O, Boulanger CM, Rautou PE. Radiology Endothelial cell-derived microparticles loaded with iron oxide nanoparticles: feasibility of MR imaging monitoring in mice. Radiology. 2012 Apr;263(1):169-78. doi: 10.1148/radiol.11111329. Epub 2012 Feb 13. PMID: 22332069.
- Alvarez-Erviti, L., Seow, Y., Yin, H., Betts, C., Lakhal, S., & Wood, M. J. a. (2011). Delivery of siRNA to the mouse brain by systemic injection of targeted exosomes. Nature Biotechnology, 29(4), 341-5. doi:10.1038/nbt.1807.
- Chargaff E, West R. The biological significance of the thromboplastic protein of blood. J Biot Chem. 1946;166: 18997.
- Deregibus MC, Cantaluppi V, Calogero R, Lo lacono M, Tetta C, Biancone L, et al. Endothelial progenitor cell derived microvesicles activate an angiogenic program in endothelial cells by a horizontal transfer of mRNA. Blood. 2007;110: 24408.
- Escudier B, Dorval T, Chaput N, André F, Caby MP, Novault S, Flament C, Leboulaire C, Borg C, Amigorena S, Boccaccio C, Bonnerot C, Dhellin O, Movassagh M, Piperno S, Robert C, Serra V, Valente N, Le Pecq JB, Spatz A, Lantz O, Tursz T, Angevin E, Zitvogel L. Vaccination of metastatic melanoma patients with autologous dendritic cell (DC) derived-exosomes: results of thefirst phase I clinical trial. J Transl Med. 2005 Mar 2;3(1):10. PMID: 15740633.
- Jiang J, Woulfe DS, Papoutsakis ET. Blood. Shear enhances thrombopoiesis and formation of microparticles that induce megakaryocytic differentiation of stem cells. 2014 Sep 25;124(13):2094-103. doi: 10.1182/blood-2014-01-547927. Epub 2014 Jun 19. PMI D: 24948658.
- Khan M, Nickoloff E, Abramova T, Johnson J, Verma SK, Krishnamurthy P, Mackie AR, Vaughan E, Garikipati VN, Benedict CL, Ramirez V, Lambers E, Ito A, Gao E, Misener S, Qin G, Luongo TS, Elrod JW, Houser SR, Koch WJ, Kishore R. Embryonic Stem Cell-Derived Exosomes Promote Endogenous Repair Mechanisms and Enhance Cardiac Function Following Myocardial Infarction. Circ Res. 2015 Apr 22. pii: CIRCRESAHA.115.305990. [Epub ahead of print] PMID: 25904597.

- Kooijmans SA, Fliervoet LA, van der Meel R, Fens MH, Heijnen HF, van Bergen En Henegouwen PM, Vader P, Schiffelers RM. PEGylated and targeted extracellular vesicles display enhanced cell specificity and circulation time. J Control Release. 2016 Feb 28;224:77-85. doi: 10.1016/j.jconrel.2016.01.009. Epub 2016 Jan 7. PMID: 26773767.
- Kooijmans SA, Stremersch S, Braeckmans K, de Smedt SC, Hendrix A, Wood MJ, Schiffelers RM, Raemdonck K, Vader P. Electroporation-induced siRNA precipitation obscures the efficiency of siRNA loading into extracellular vesicles J Control Release. 2013;172(1):229-38.
- Haney MJ, Klyachko NL, Zhao Y, Gupta R, Plotnikova EG, He Z, Patel T, Piroyan A, Sokolsky M, Kabanov AV, Batrakova EV.Exosomes as drug delivery vehicles for Parkinson's disease therapy. J Control Release. 2015 Jun 10;207:18-30. doi: 10.1016/j.jconrel.2015.03.033. Epub 2015 Mar 31. PubMed PMID: 25836593; PubMed Central PMCID: PMC4430381.
- Lamichhane TN, Raiker RS, Jay SM. Exogenous DNA Loading into Extracellular Vesicles via Electroporation is Size-Dependent and Enables Limited Gene Delivery. Mol Pharm. 2015 Oct 5;12(10):3650-7. doi: 10.1021/acs.molpharmaceut.5b00364. Epub 2015 Sep 23. PMID: 26376343.
- Massart R. Preparation of Aqueous Magnetic Liquids in Alkaline and Acidic Media. IEEE Trans Magn. 1981;17:1247-8.
- Parlati F, Weber T, McNew JA, Westermann B, Söllner TH, Rothman JE. Rapid and efficient fusion of phospholipid vesicles by the alpha-helical core of a SNARE complex in the absence of an N-terminal regulatory domain. Proc Natl Acad Sci USA. 1999 Oct 26;96(22):12565-70. PMID: 10535962.
- Ridder K, Keller S, Dams M, Rupp AK, Schlaudraff J, Turco DD, Starmann J, Macas J, Karpova D, Devraj K, Depboylu C, Landfried B, Arnold B, Plate KH, Höglinger G, Sültmann H, Altevogt P, Momma S. Extracellular vesicle-mediated transfer of genetic information between the hematopoietic system and the brain in response to inflammation. PLoS Biol. 2014 Jun 3;12(6):e1001874. doi: 10.1371/journal.pbio.1001874. eCollection 2014 Jun. PMID: 24893313.
- Sato YT, Umezaki K, Sawada S, Mukai SA, Sasaki Y, Harada N, Shiku H, Akiyoshi K. Engineering hybrid exosomes by membrane fusion with liposomes. Sci Rep. 2016 Feb 25;6:21933. doi: 10.1038/srep21933. PubMed PMID: 26911358; PubMed Central PMCID: PMC4766490.Scott BL, Van Komen JS, Liu S, Weber T, Melia TJ, McNew JA. Liposome fusion assay to monitor intracellular membrane fusion machines. Methods Enzymol. 2003;372:274-300. No abstract available. PMID: 14610819.
- Shen Y, Torchia MLG, Lawson GW, Karp CL, Ashwell JD, Mazmanian SK. Outer membrane vesicles of a human commensal mediate immune regulation and disease protection. Cell Host Microbe. 2012; 12:509-20. [PubMed: 22999859]
- Shtam TA, Kovalev RA, Varfolomeeva EY, Makarov EM, Kil YV, Filatov MV. Exosomes are natural carriers of exogenous siRNA to human cells in vitro. Cell Commun Signal. 2013 Nov 18;11:88. doi: 10.1186/1478-811X-11-88. PMID: 24245560.
- Smyth T, Petrova K, Payton NM, Persaud I, Redzic JS, Graner MW, Smith-Jones P, Anchordoquy TJ. Surface functionalization of exosomes using click chemistry. Bioconjug Chem. 2014 Oct 15;25(10):1777-84. doi: 10.1021/bc500291r. Epub 2014 Sep 30. PubMed PMID: 25220352; PubMed Central PMCID: PMC4198107.
- Thery C, Amigorena S, Raposo G, Clayton A. Isolation and characterization of exosomes from cell culturesupernatants and biological fluids. Curr Protoc Cell Biol. 2006 Apr;Chapter 3:Unit 3.22. doi: 10.1002/0471143030.cb0322s30. PMID: 18228490.

Tian Y, Li S, Song J, Ji T, Zhu M, et al. A doxorubicin delivery platform using engineered natural membrane vesicle exosomes for targeted tumor therapy. Biomaterials. 2014; 35:2383-90. [PubMed: 24345736].


**Claims**

1. An *in vitro* method for obtaining a biohybrid vector comprising the step of contacting at least one extracellular vesicle (EV) with at least one liposome containing a compound of interest, wherein a fusion between the extracellular vesicle and the liposome is performed in the presence of polyethylene glycol (PEG).

2. The method of claims 1 further comprising a step of isolating the non-fused extracellular vesicles and liposomes and/or the biohybrid vectors.

3. The method of claims 1 or 2, wherein the extracellular vesicle is one selected from the group comprising a microvesicle, an exosome and an apoptic body.

4. The method according to any one of claims 1 to 3, wherein the fusion is performed at temperature between 30°C and 70°C, preferably at 40°C.

**5.** The method according to any one of claims 1 to 4, wherein the compound of interest is at least one selected from the group comprising a nanoparticle, a supramolecular assembly, a hydrophobic molecule, a hydrophilic molecule and an amphiphilic molecule.

**6.** The method of claim 5, wherein the compound of interest is selected from the group comprising a hydrophobic molecule selected from the group comprising waxes, fats, oils, alkanes, membrane proteins , a hydrophilic molecule selected from the group comprising colloids, polar covalent compounds like alcohols such as $C_2H_5OH$ (ethanol) and ketones like $(CH_3)_2C=O$ (acetone), sugars, ionic compounds like KCl, nucleic acids, amino acids, phosphate esters, calcium chloride, $CaCl_2$, non steroid hormones, glucose, insulin, mineral acids and bases and an amphiphilic molecule selected from the group comprising phospholipids, glycolipids, cholesterol, pepducins, fatty acids, bile acids, saponins, local anaesthetics.

**7.** The method of claim 5 or 6, wherein the compound of interest is a hydrophilic compound selected from the group comprising siRNA, miRNA, DNA, proteins or nanoparticle selected from the group comprising solid lipid nanoparticles, polymeric particles, metal nanoparticles, nanotubes, iron oxide nanoparticles, gold nanoparticles, multi-layer nanoparticles, dendrimers, quantum dots

**8.** The method according to any one of claims 1 to 7, wherein the liposome contains a lipid medium comprising polar lipids preferably the polar lipids selected from the group comprising phosphatidyl ethanolamine POPC, DOPC, $DOP_{12}$-biotin, DOPS-NBD, $DOP_{13(orB)-}$Rho, $DOP_{13}$-NBO or non polar lipid.

**9.** The method according to any one of claims 1 to 8, wherein PEG have a molecular mass comprised between 3000 g/mol and 15000 g/mol, which is preferably 8000 g/mol.

**10.** The method according to any one of claims 1 to 8, wherein PEG have a concentration comprised between 2% w/v and 60% w/v, preferably between 5% w/v and 40% w/v and more preferably 20% w/v of the total weight of all reacting compounds.

**11.** A biohybrid vector obtaining by the method of anyone of claims 1 to 10 which consists of an extracellular vesicle fused with a liposome containing a compound of interest.

**12.** The biohybrid vector of claim 11 for use as drug.

**13.** The biohybrid vector of claim 12 for use as drug for treating disease selected from immune diseases, degenerative diseases, metabolic disease, infectious diseases and cancer.

**14.** The biohybrid vector of claim 12, for use as vaccine.

**15.** The biohybrid vector according to claim 11 for use in medical imaging.

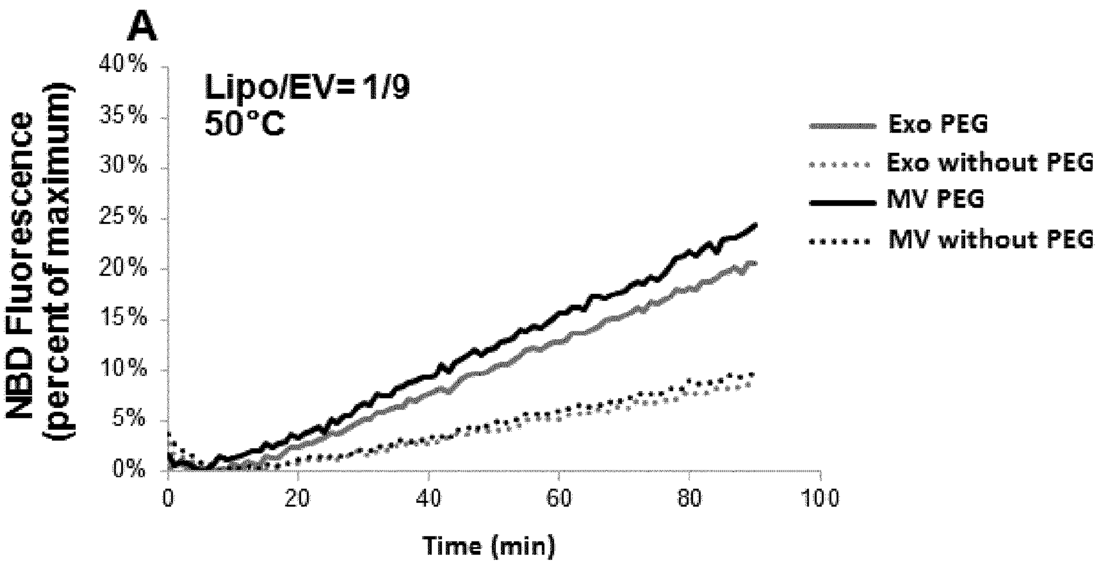
A
Lipo/EV= 1/9
50°C
NBD Fluorescence (percent of maximum)
40%
35%
30%
25%
20%
15%
10%
5%
0%
0
20
40
60
80
100
Time (min)
Exo PEG
Exo without PEG
MV PEG
MV without PEG

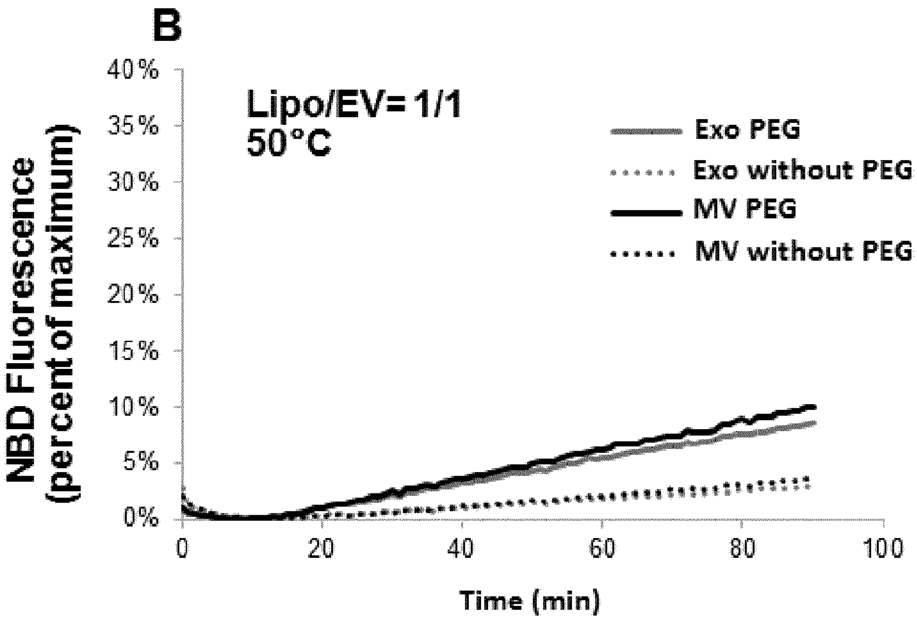
B
Lipo/EV= 1/1
50°C
NBD Fluorescence (percent of maximum)
40%
35%
30%
25%
20%
15%
10%
5%
0%
0
20
40
60
80
100
Time (min)
Exo PEG
Exo without PEG
MV PEG
MV without PEG

Fig. 1

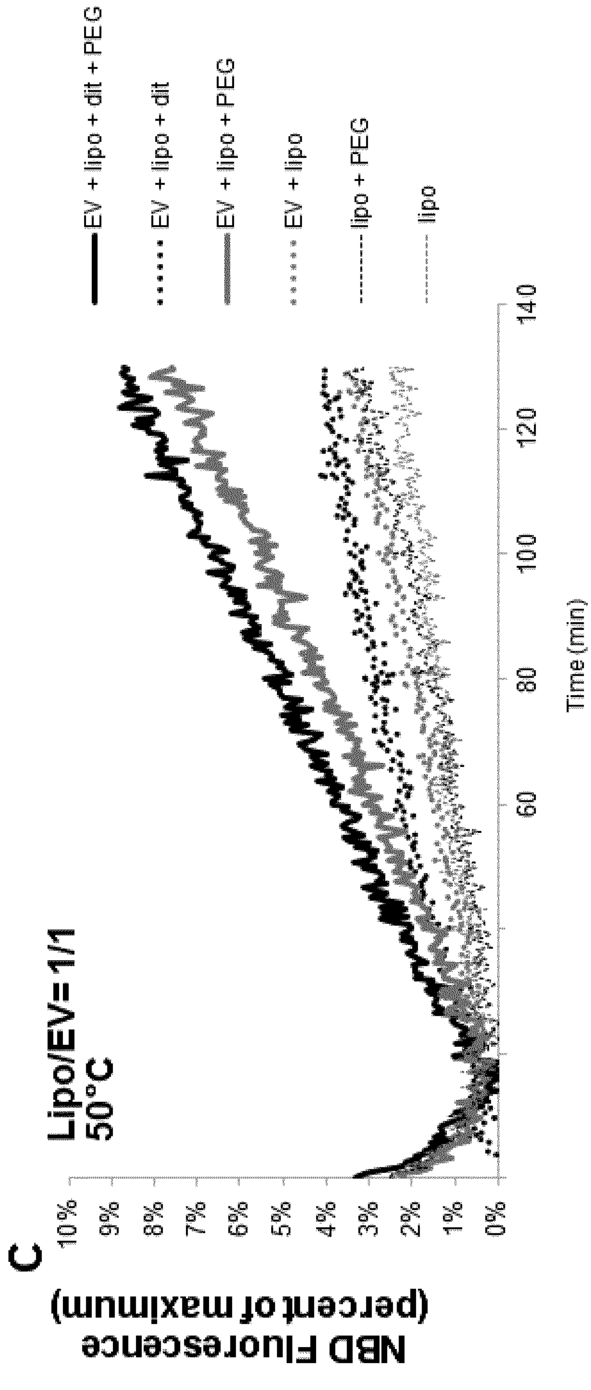


Fig. 1 (continued)

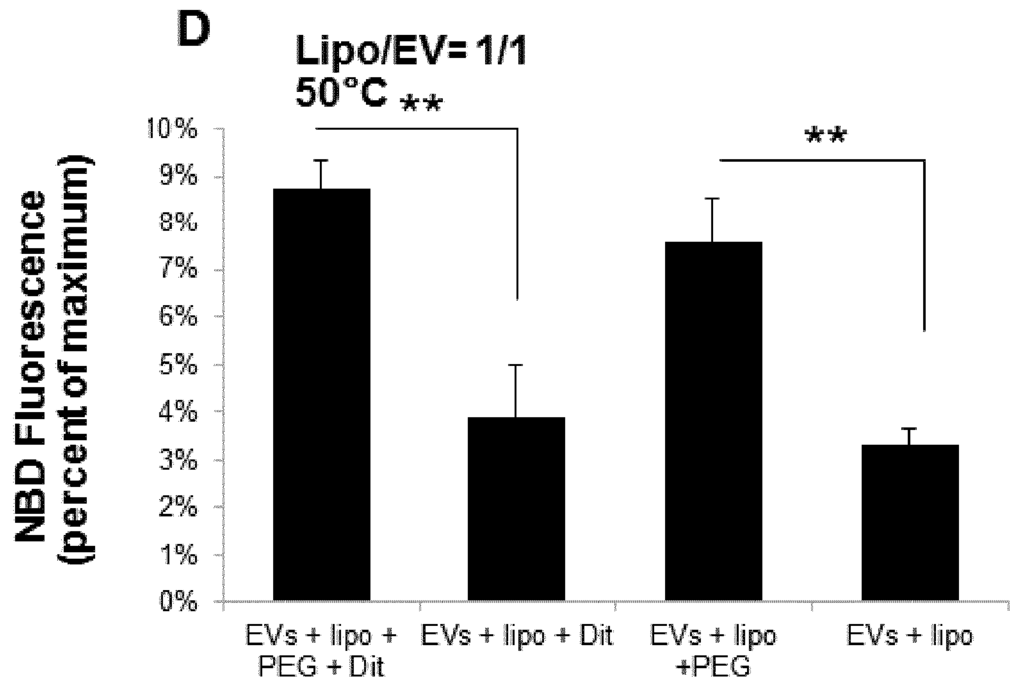


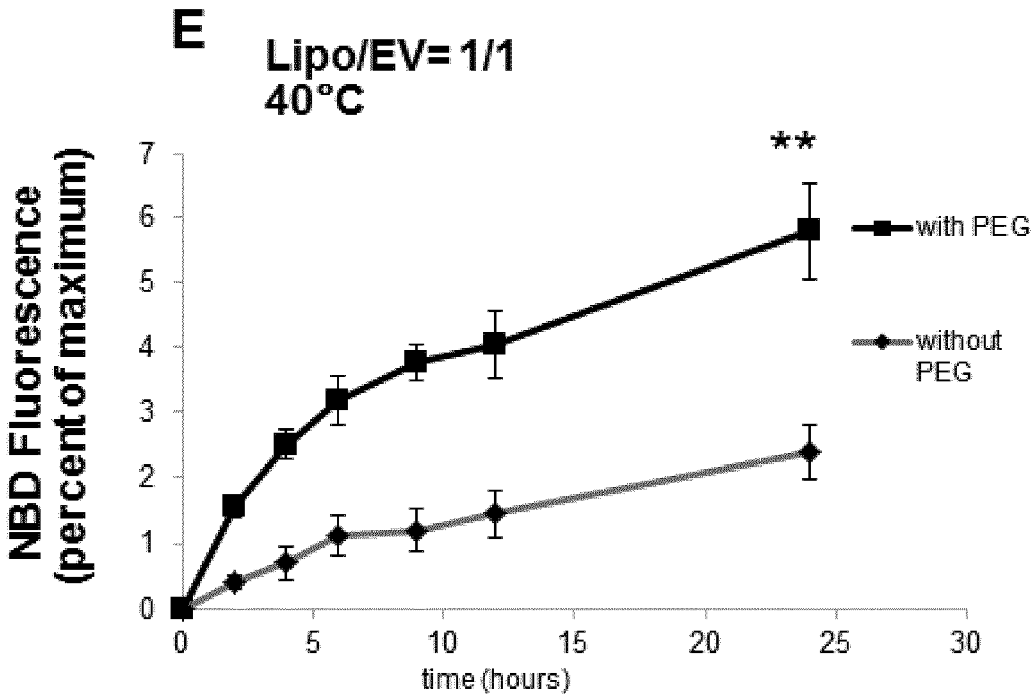


Fig. 1 (continued)

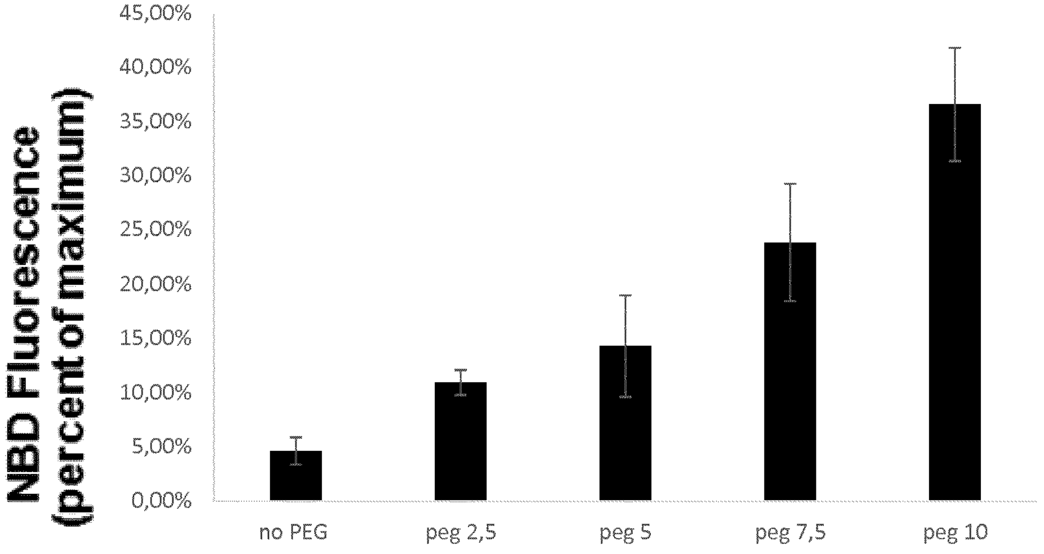
NBD Fluorescence (percent of maximum)
45,00%
40,00%
35,00%
30,00%
25,00%
20,00%
15,00%
10,00%
5,00%
0,00%
no PEG
peg 2,5
peg 5
peg 7,5
peg 10

Fig. 2

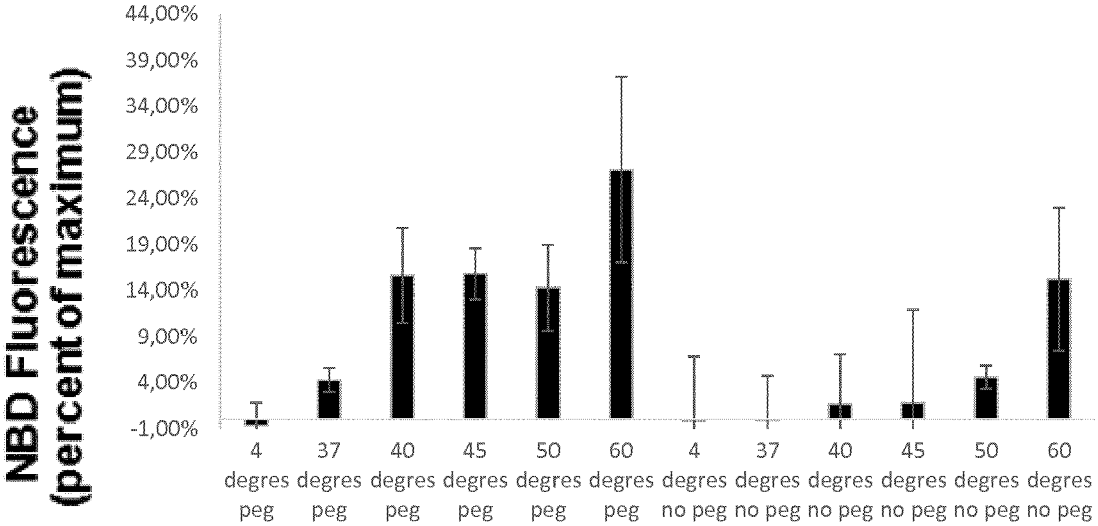
NBD Fluorescence (percent of maximum)
44,00%
39,00%
34,00%
29,00%
24,00%
19,00%
14,00%
9,00%
4,00%
-1,00%
4 degres peg
37 degres peg
40 degres peg
45 degres peg
50 degres peg
60 degres peg
4 degres no peg
37 degres no peg
40 degres no peg
45 degres no peg
50 degres no peg
60 degres no peg

Fig. 3

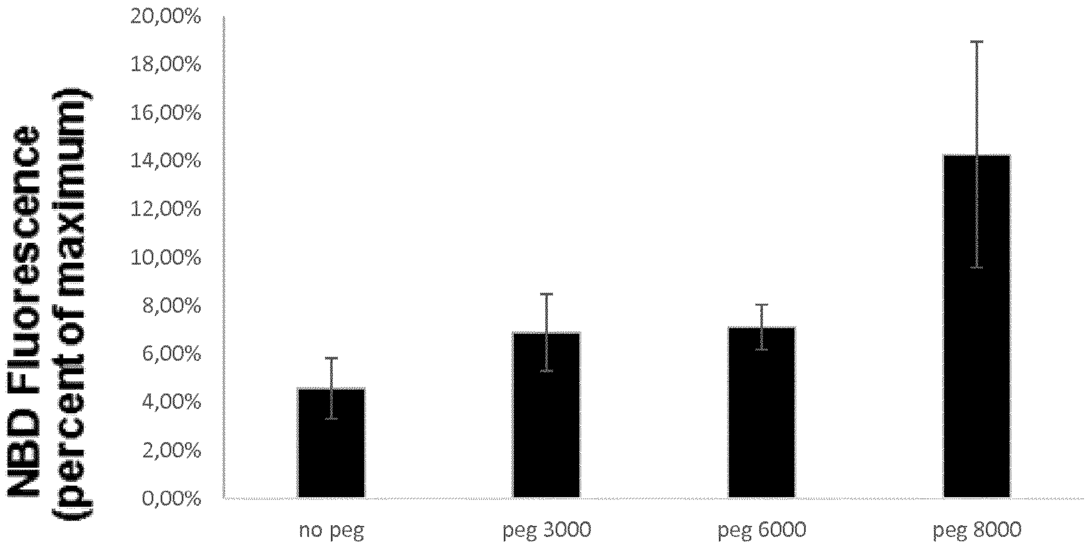
NBD Fluorescence
(percent of maximum)
20,00%
18,00%
16,00%
14,00%
12,00%
10,00%
8,00%
6,00%
4,00%
2,00%
0,00%
no peg
peg 3000
peg 6000
peg 8000

Fig. 4

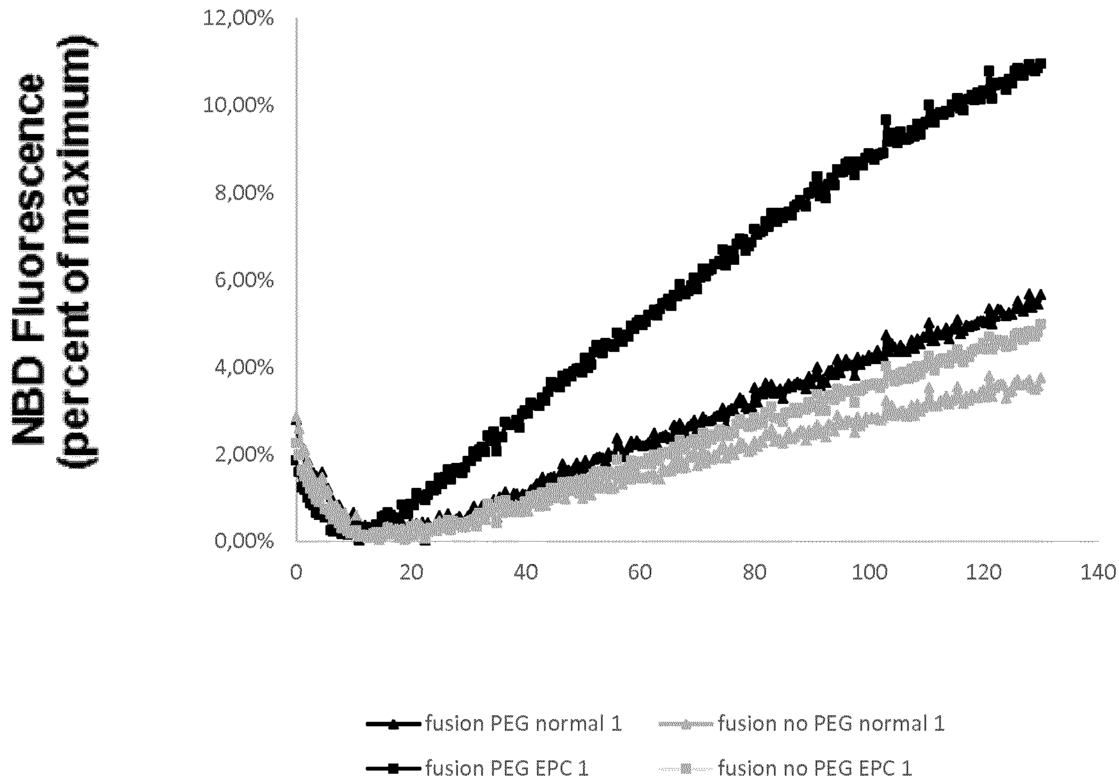
NBD Fluorescence
(percent of maximum)
12,00%
10,00%
8,00%
6,00%
4,00%
2,00%
0,00%
0
20
40
60
80
100
120
140
fusion PEG normal 1
fusion no PEG normal 1
fusion PEG EPC 1
fusion no PEG EPC 1

Fig. 5

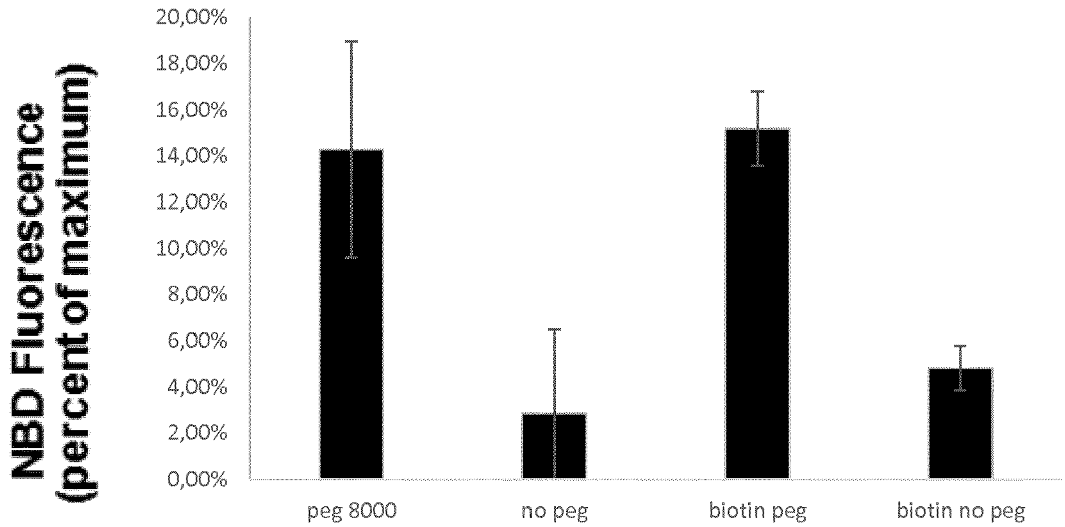
NBD Fluorescence (percent of maximum)
20,00%
18,00%
16,00%
14,00%
12,00%
10,00%
8,00%
6,00%
4,00%
2,00%
0,00%
peg 8000
no peg
biotin peg
biotin no peg

Fig. 6

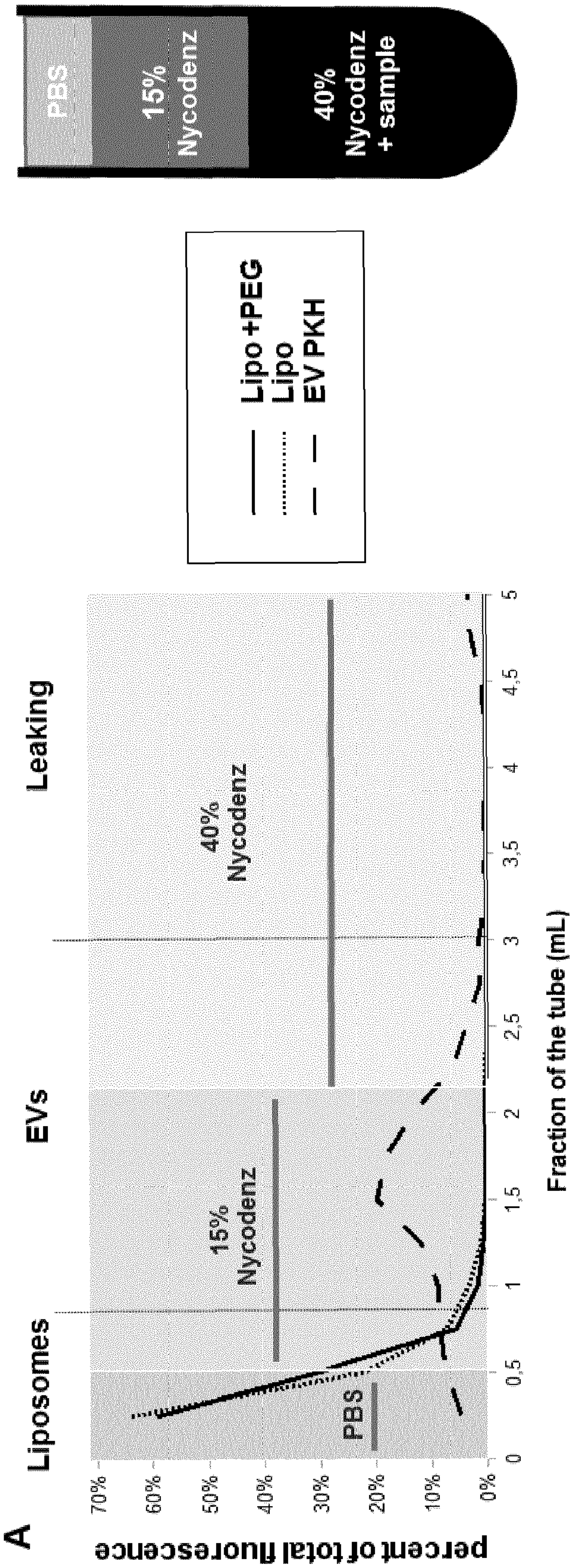
A
percent of total fluorescence
70%
60%
50%
40%
30%
20%
10%
0%
0
0,5
1
1,5
2
2,5
3
3,5
4
4,5
5
Fraction of the tube (mL)
Liposomes
EVs
Leaking
PBS
15%
Nycodenz
40%
Nycodenz
Lipo +PEG
Lipo
EV PKH
PBS
15%
Nycodenz
40%
Nycodenz
+ sample

Fig. 7

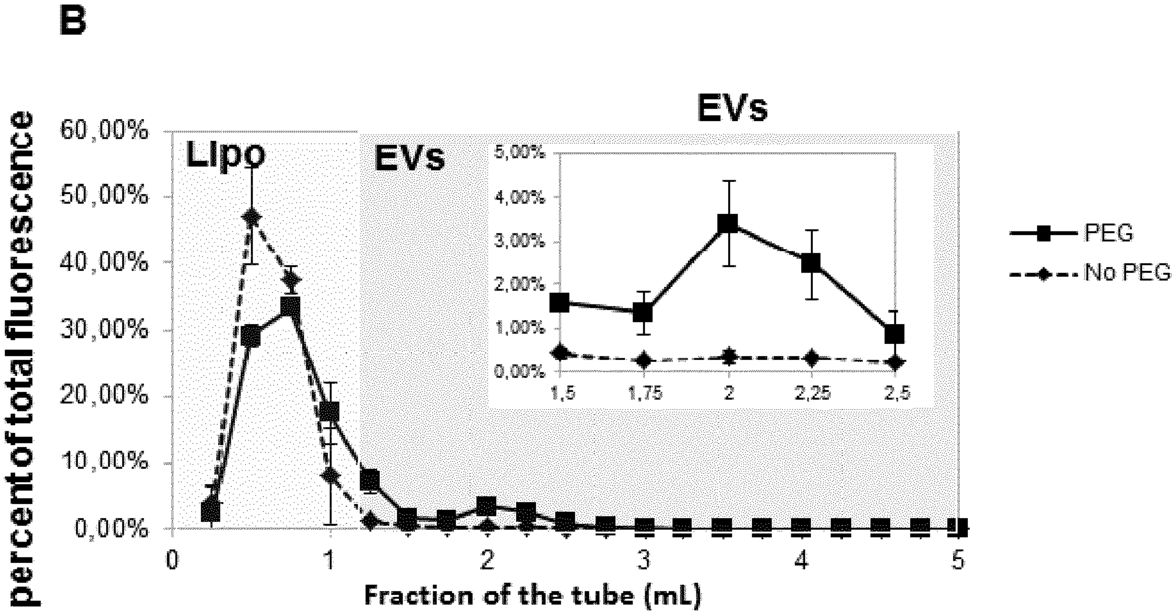


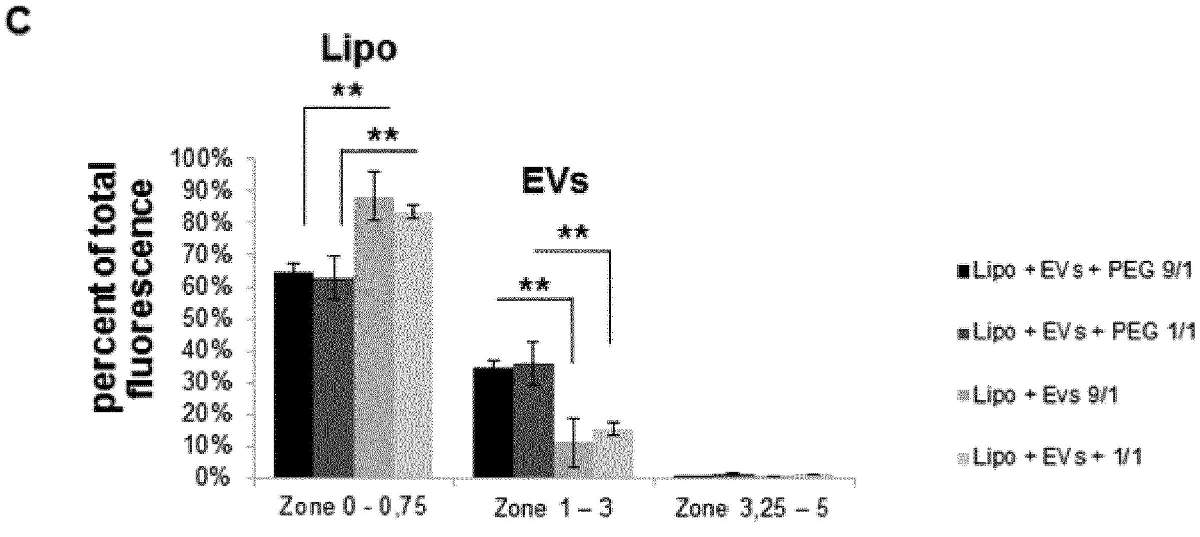


Fig. 7 (continued)

D

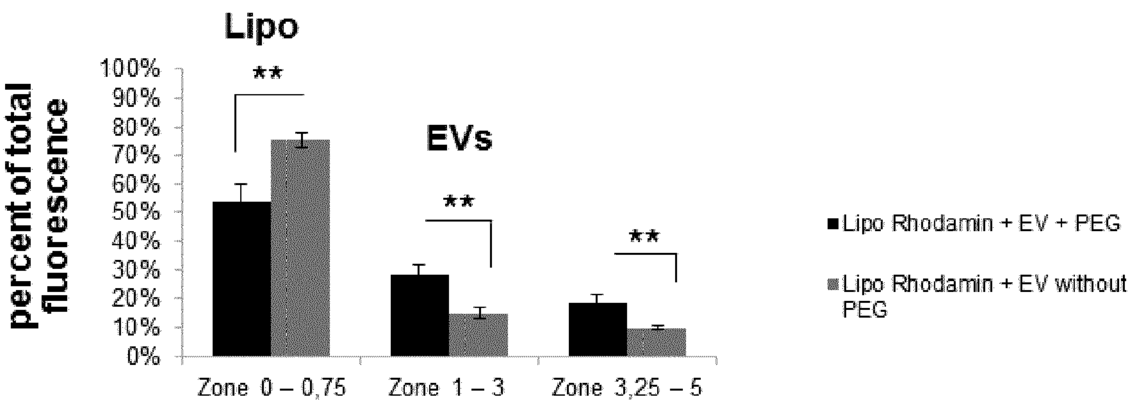


E

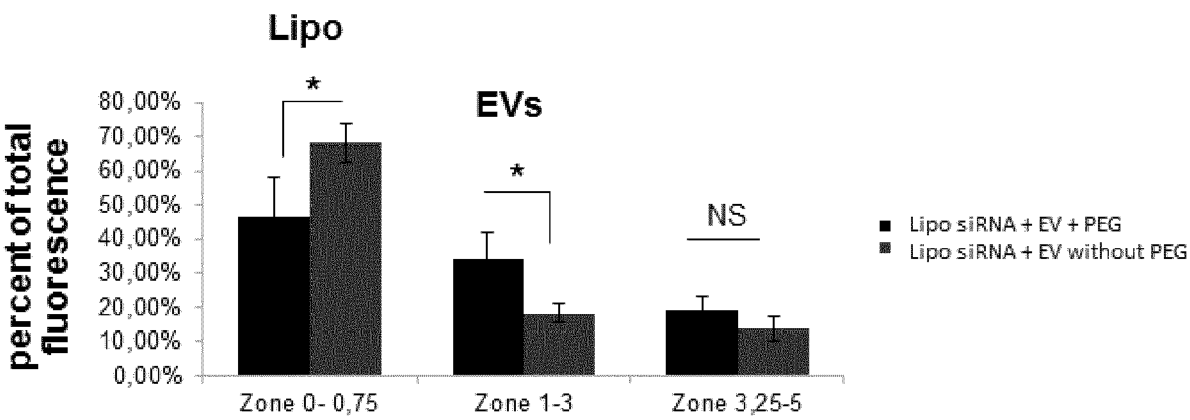


Fig. 7 (continued)

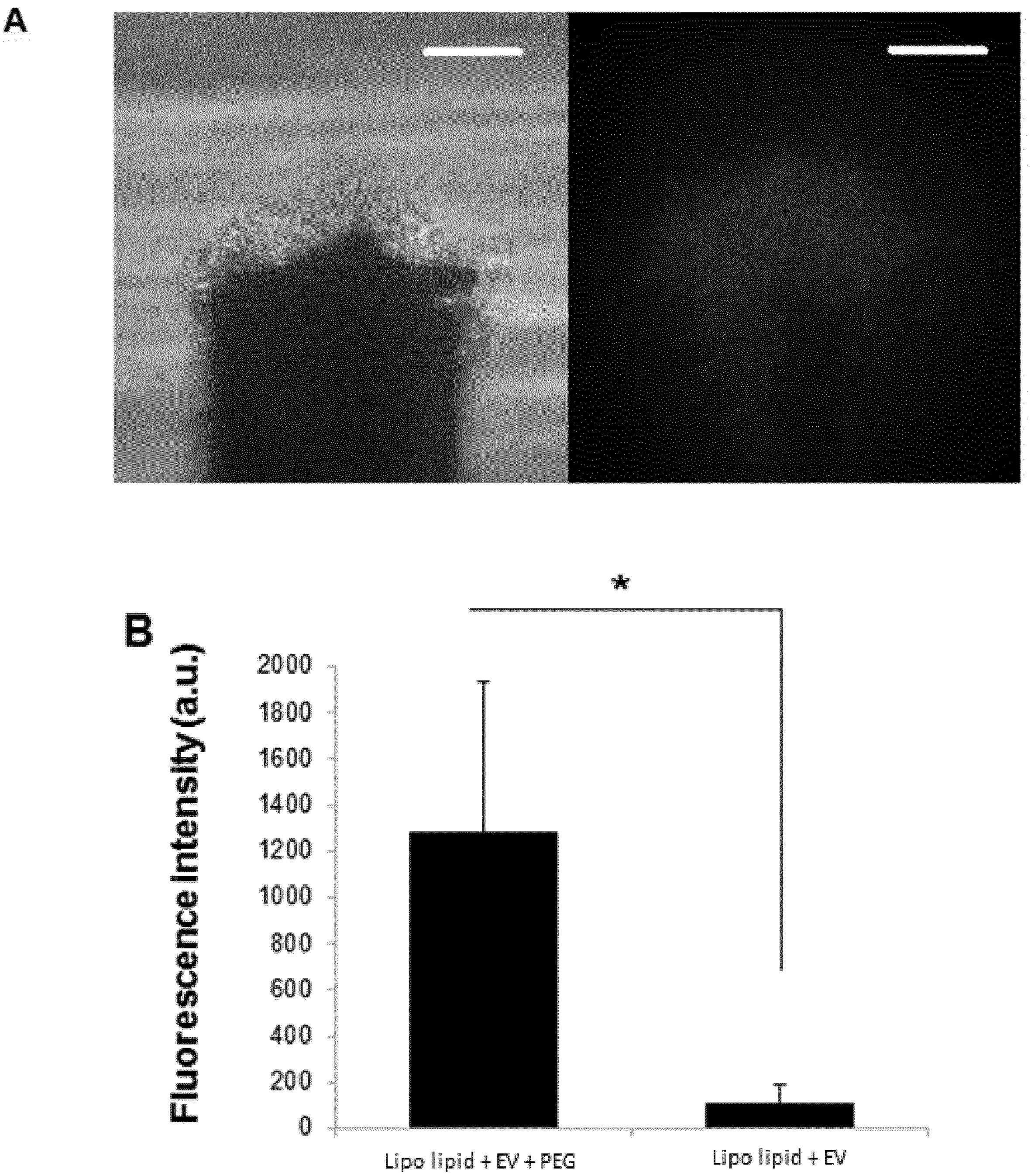
A
B
*
Fluorescence Intensity (a.u.)
2000
1800
1600
1400
1200
1000
800
600
400
200
0
Lipo lipid + EV + PEG
Lipo lipid + EV

Fig. 8

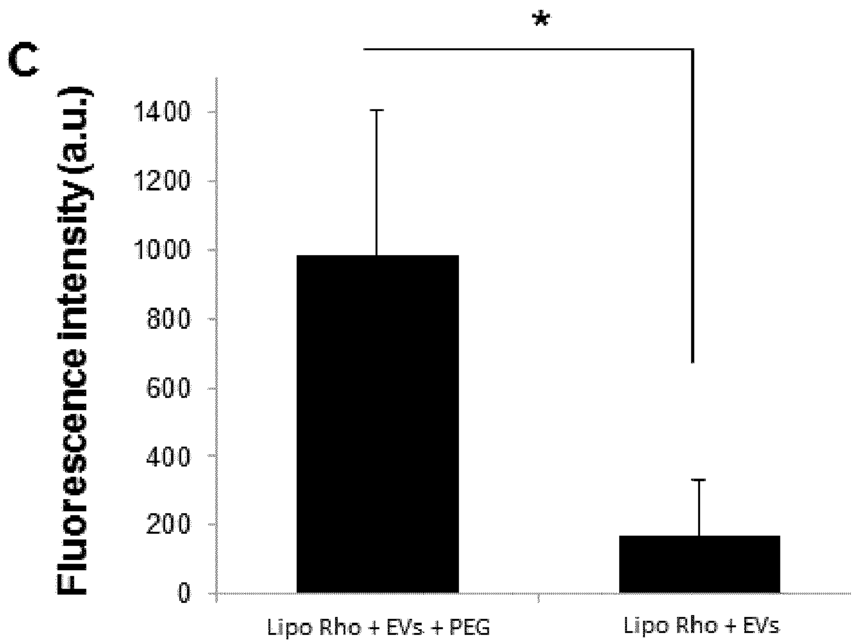


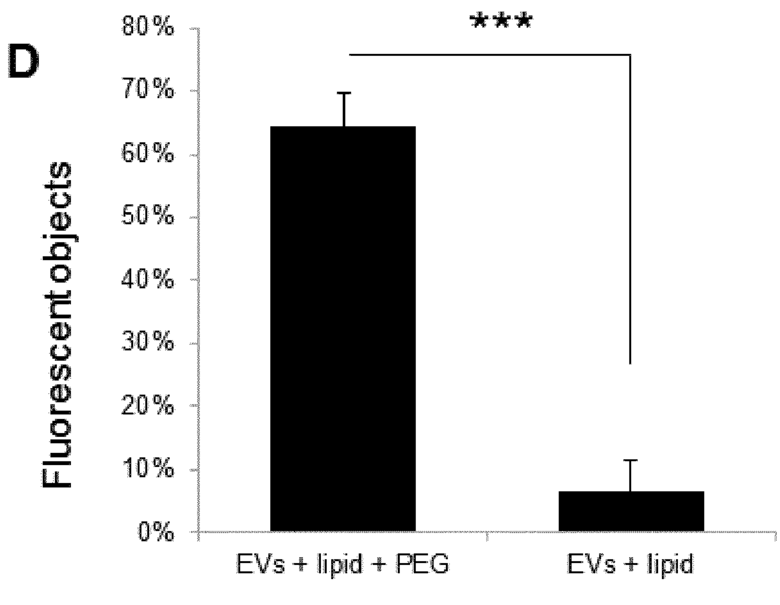


Fig. 8 (continued)

E

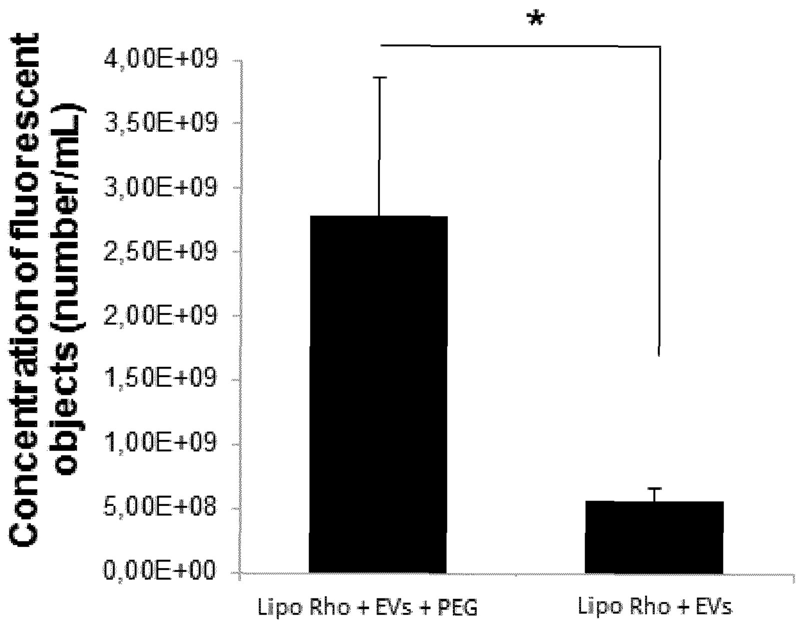


Fig. 8 (continued)

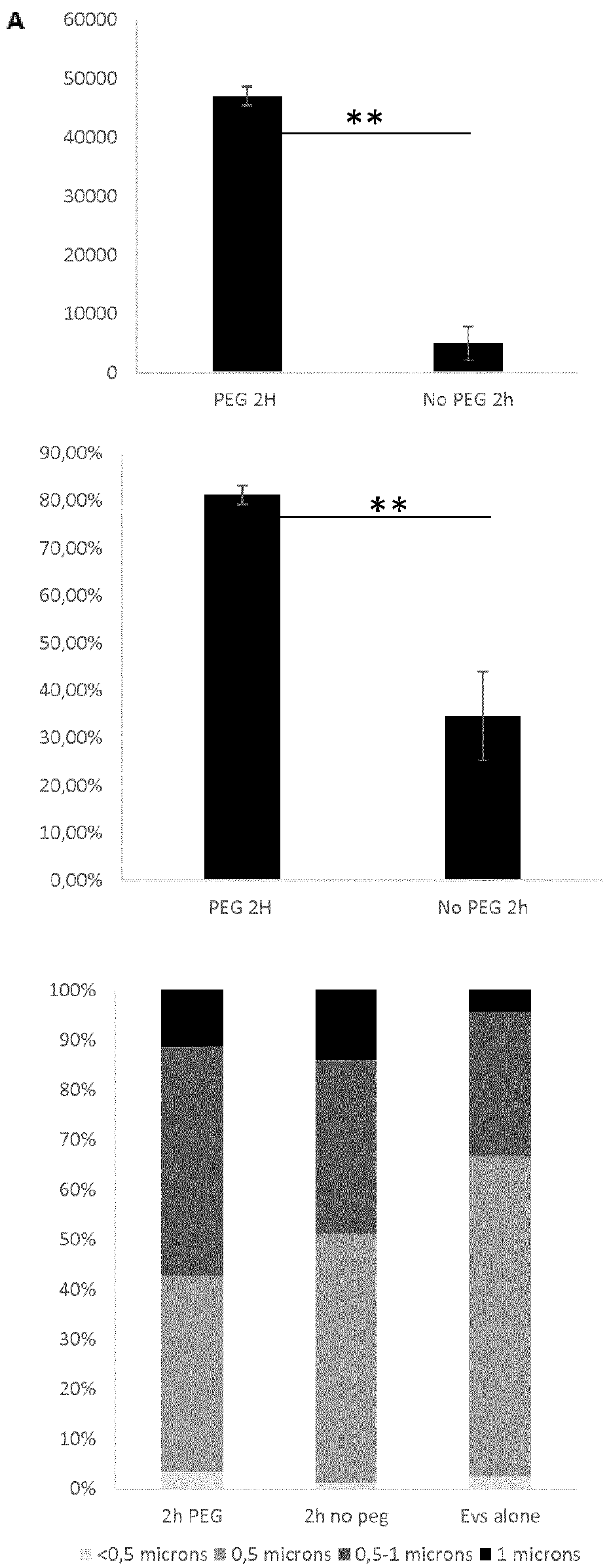
A
60000
50000
40000
30000
20000
10000
0
**
PEG 2H
No PEG 2h
B
90,00%
80,00%
70,00%
60,00%
50,00%
40,00%
30,00%
20,00%
10,00%
0,00%
**
PEG 2H
No PEG 2h
C
100%
90%
80%
70%
60%
50%
40%
30%
20%
10%
0%
2h PEG
2h no peg
Evs alone
<0,5 microns
0,5 microns
0,5-1 microns
1 microns

Fig. 9

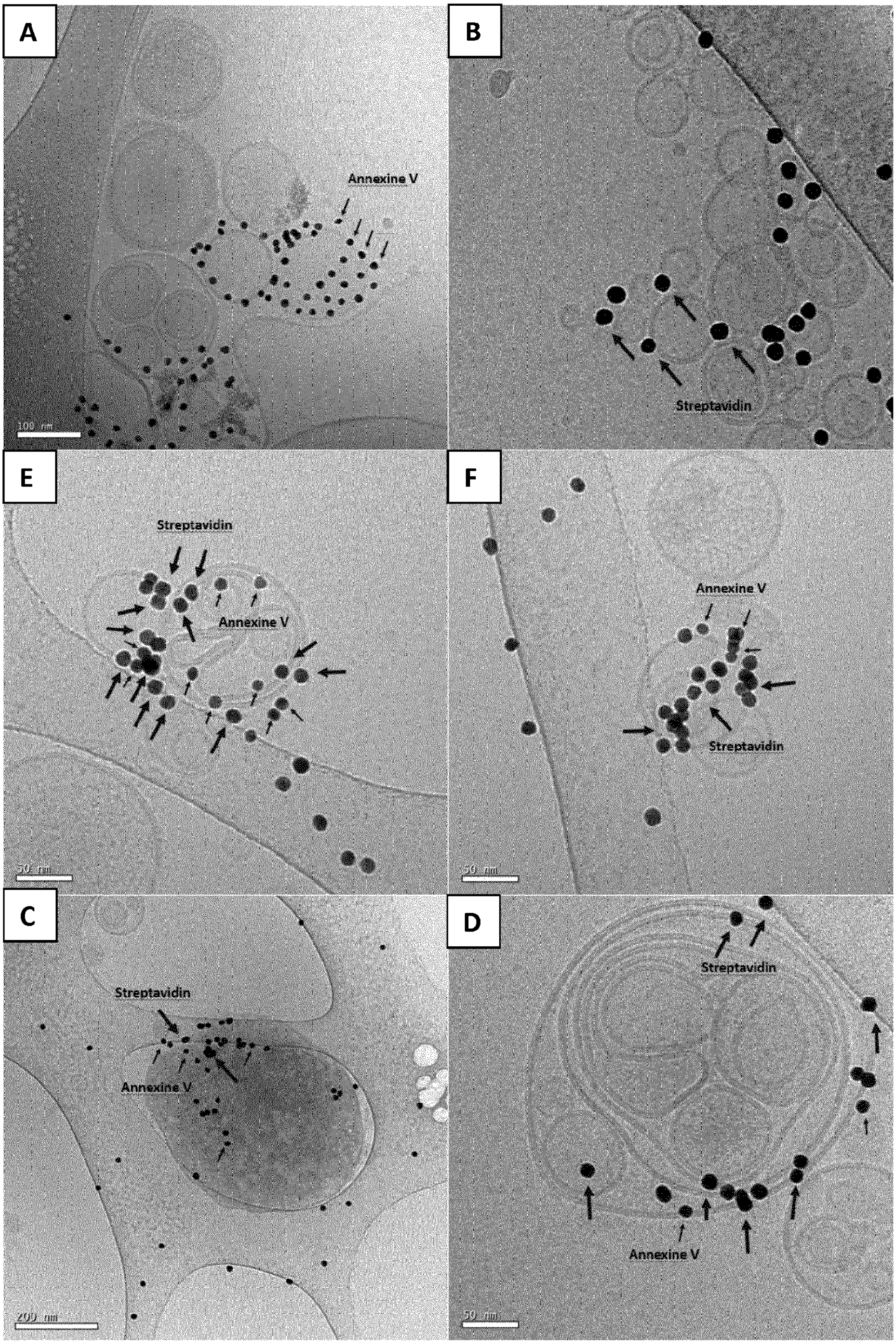
A
Annexine V
100 nm
B
Streptavidin
E
Streptavidin
Annexine V
50 nm
F
Annexine V
Streptavidin
50 nm
C
Streptavidin
Annexine V
200 nm
D
Streptavidin
Annexine V
50 nm

Fig. 10

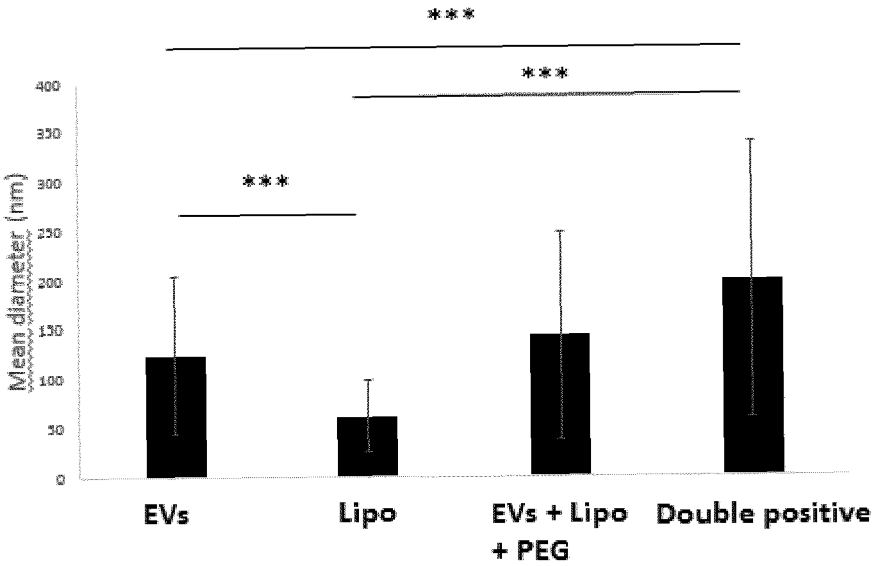
***
***
***
400
350
300
250
200
150
100
50
0
Mean diameter (nm)
EVs
Lipo
EVs + Lipo + PEG
Double positive

Fig. 11

Europäisches Patentamt
European Patent Office
Office européen des brevets

# EUROPEAN SEARCH REPORT

Application Number
EP 16 30 6163

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/110957 A2 (DE BEER JOEL [CH]) 30 July 2015 (2015-07-30)<br>* page 1, line 5 - page 14, line 9 *<br>* page 24, line 11 - page 26, line 12 *<br>* page 48, line 16 - page 71, line 11; examples 1-13 *<br>* claims 1-4, 9, 20, 24, 26-31 *<br>----- | 1-15 | INV.<br>A61K9/127<br>A61K9/50<br>A61K9/51 |
| X | JP 2014 185090 A (UNIV KYOTO) 2 October 2014 (2014-10-02)<br>* paragraph [0001] *<br>* paragraph [0010] - paragraph [0073] *<br>* claims 1-9 *<br>----- | 1-15 | |
| X | KR 101 585 611 B1 (KOREA ADVANCED INST SCI & TECH [KR]) 15 January 2016 (2016-01-15)<br>* paragraph [0007] - paragraph [0121] *<br>* claims 1, 6, 8 *<br>----- | 1-15 | |

TECHNICAL FIELDS SEARCHED (IPC)
A61K

The present search report has been drawn up for all claims

1

| Place of search | Date of completion of the search | Examiner |
|---|---|---|
| The Hague | 14 March 2017 | González Ferreiro, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 30 6163

This annex lists the patent family members relating to the patent documents cited in the above-mentioned European search report.
The members are as contained in the European Patent Office EDP file on
The European Patent Office is in no way liable for these particulars which are merely given for the purpose of information.

14-03-2017

| Patent document cited in search report | Publication date | Patent family member(s) | Publication date |
|---|---|---|---|
| WO 2015110957 A2 | 30-07-2015 | AU 2015208837 A1 | 11-08-2016 |
| | | CA 2936514 A1 | 30-07-2015 |
| | | CN 105934240 A | 07-09-2016 |
| | | EA 201691448 A1 | 30-12-2016 |
| | | EP 3096741 A2 | 30-11-2016 |
| | | JP 2017502997 A | 26-01-2017 |
| | | KR 20160110410 A | 21-09-2016 |
| | | US 2016354313 A1 | 08-12-2016 |
| | | WO 2015110957 A2 | 30-07-2015 |
| JP 2014185090 A | 02-10-2014 | NONE | |
| KR 101585611 B1 | 15-01-2016 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Radiology,* April 2012, vol. 263 (1), 169-78
- **ALVAREZ-ERVITI, L. ; SEOW, Y. ; YIN, H. ; BETTS, C. ; LAKHAL, S. ; WOOD, M. J.** Delivery of siRNA to the mouse brain by systemic injection of targeted exosomes. *Nature Biotechnology,* 2011, vol. 29 (4), 341-5
- **CHARGAFF E ; WEST R.** The biological significance of the thromboplastic protein of blood. *J Biot Chem.,* 1946, vol. 166, 18997
- **DEREGIBUS MC ; CANTALUPPI V ; CALOGERO R ; LO LACONO M ; TETTA C ; BIANCONE L et al.** Endothelial progenitor cell derived microvesicles activate an angiogenic program in endothelial cells by a horizontal transfer of mRNA. *Blood,* 2007, vol. 110, 24408
- **ESCUDIER B ; DORVAL T ; CHAPUT N ; ANDRÉ F ; CABY MP ; NOVAULT S ; FLAMENT C ; LEBOULAIRE C ; BORG C ; AMIGORENA S.** Vaccination of metastatic melanoma patients with autologous dendritic cell (DC) derived-exosomes: results of thefirst phase I clinical trial. *J Transl Med.,* 02 March 2005, vol. 3 (1), 10
- **JIANG J ; WOULFE DS ; PAPOUTSAKIS ET.** Shear enhances thrombopoiesis and formation of microparticles that induce megakaryocytic differentiation of stem cells. *Blood,* 25 September 2014, vol. 124 (13), 2094-103
- **KHAN M ; NICKOLOFF E ; ABRAMOVA T ; JOHNSON J ; VERMA SK ; KRISHNAMURTHY P ; MACKIE AR ; VAUGHAN E ; GARIKIPATI VN ; BENEDICT CL.** Embryonic Stem Cell-Derived Exosomes Promote Endogenous Repair Mechanisms and Enhance Cardiac Function Following Myocardial Infarction. *Circ Res.,* 22 April 2015
- **KOOIJMANS SA ; FLIERVOET LA ; VAN DER MEEL R ; FENS MH ; HEIJNEN HF ; VAN BERGEN EN ; HENEGOUWEN PM ; VADER P ; SCHIFFELERS RM.** PEGylated and targeted extracellular vesicles display enhanced cell specificity and circulation time. *J Control Release,* 28 February 2016, vol. 224, 77-85
- **KOOIJMANS SA ; STREMERSCH S ; BRAECKMANS K ; DE SMEDT SC ; HENDRIX A ; WOOD MJ ; SCHIFFELERS RM ; RAEMDONCK K ; VADER P.** Electroporation-induced siRNA precipitation obscures the efficiency of siRNA loading into extracellular vesicles. *J Control Release,* 2013, vol. 172 (1), 229-38
- **HANEY MJ ; KLYACHKO NL ; ZHAO Y ; GUPTA R ; PLOTNIKOVA EG ; HE Z ; PATEL T ; PIROYAN A ; SOKOLSKY M ; KABANOV AV.** Exosomes as drug delivery vehicles for Parkinson's disease therapy. *J Control Release,* 10 June 2015, vol. 207, 18-30
- **LAMICHHANE TN ; RAIKER RS ; JAY SM.** Exogenous DNA Loading into Extracellular Vesicles via Electroporation is Size-Dependent and Enables Limited Gene Delivery. *Mol Pharm.,* 05 October 2015, vol. 12 (10), 3650-7
- **MASSART R.** Preparation of Aqueous Magnetic Liquids in Alkaline and Acidic Media. *IEEE Trans Magn.,* 1981, vol. 17, 1247-8
- **PARLATI F ; WEBER T ; MCNEW JA ; WESTERMANN B ; SÖLLNER TH ; ROTHMAN JE.** Rapid and efficient fusion of phospholipid vesicles by the alpha-helical core of a SNARE complex in the absence of an N-terminal regulatory domain. *Proc Natl Acad Sci USA.,* 26 October 1999, vol. 96 (22), 12565-70
- **RIDDER K ; KELLER S ; DAMS M ; RUPP AK ; SCHLAUDRAFF J ; TURCO DD ; STARMANN J ; MACAS J ; KARPOVA D ; DEVRAJ K.** Extracellular vesicle-mediated transfer of genetic information between the hematopoietic system and the brain in response to inflammation. *PLoS Biol.,* 03 June 2014, vol. 12 (6), e1001874
- **SATO YT ; UMEZAKI K ; SAWADA S ; MUKAI SA ; SASAKI Y ; HARADA N ; SHIKU H ; AKIYOSHI K.** Engineering hybrid exosomes by membrane fusion with liposomes. *Sci Rep.,* 25 February 2016, vol. 6, 21933
- **SCOTT BL ; VAN KOMEN JS ; LIU S ; WEBER T ; MELIA TJ ; MCNEW JA.** Liposome fusion assay to monitor intracellular membrane fusion machines. *Methods Enzymol.,* 2003, vol. 372, 274-300
- **SHEN Y ; TORCHIA MLG ; LAWSON GW ; KARP CL ; ASHWELL JD ; MAZMANIAN SK.** Outer membrane vesicles of a human commensal mediate immune regulation and disease protection. *Cell Host Microbe,* 2012, vol. 12, 509-20
- **SHTAM TA ; KOVALEV RA ; VARFOLOMEEVA EY ; MAKAROV EM ; KIL YV ; FILATOV MV.** Exosomes are natural carriers of exogenous siRNA to human cells in vitro. *Cell Commun Signal,* 18 November 2013, vol. 11, 88

- **SMYTH T ; PETROVA K ; PAYTON NM ; PERSAUD I ; REDZIC JS ; GRANER MW ; SMITH-JONES P ; ANCHORDOQUY TJ.** Surface functionalization of exosomes using click chemistry. *Bioconjug Chem.,* 15 October 2014, vol. 25 (10), 1777-84
- **THERY C ; AMIGORENA S ; RAPOSO G ; CLAYTON A.** Isolation and characterization of exosomes from cell culturesupernatants and biological fluids. *Curr Protoc Cell Biol.,* April 2006
- **TIAN Y ; LI S ; SONG J ; JI T ; ZHU M et al.** A doxorubicin delivery platform using engineered natural membrane vesicle exosomes for targeted tumor therapy. *Biomaterials,* 2014, vol. 35, 2383-90